# EUROPEAN PATENT APPLICATION

(11) **EP 3 235 513 A1**
(43) Date of publication of application: **25.10.2017**
(21) Application number: 16165764.8
(22) Date of filing: 18.04.2016
(51) Int. Cl.: A61K 47/50, A61K 48/00, C12N 15/87

(54) **IMPROVEMENTS IN OR RELATING TO ORGANIC MATERIAL**

(71) Applicant: Université de Liège, 4000 Liège (BE); Université Paris 6 Pierre et Marie Curie UPMC, 75005 Paris (FR); Centre National de la Recherche Scientifique (C.N.R.S.), 75016 Paris (FR)
(72) Inventor: Debuigne, Antoine, 4000 Liège (BE); Dréan, Mathilde, 4030 Grivégnée (BE); Guégan, Philippe, 75252 Paris cedex 05 (FR); Jérôme, Christine, 4000 Liège (BE); Midoux, Patrick, 45071 Orléans (FR); Rieger, Jutta, 75252 Paris cedex 05 (FR)
(74) Representative: Peel, James Peter

(57) **Abstract**

The present invention provides polyplex comprising:
(a) at least one polymer of formula (I) comprising at least one set of repeating units of formula: 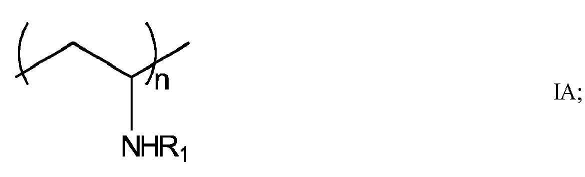 and 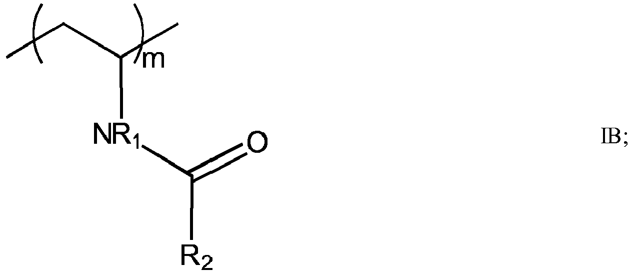 wherein for each set of repeating units R₁ represents either a hydrogen atom or a straight or branched chain alkyl group having from 1 to 20 carbon atoms and R₂ represents a straight or branched chain alkyl group having from 1 to 20 carbon atoms,
wherein n and m each represent an integer of from 1 to 5000,
wherein the sum of n and m is from 10 to 250 when R₁ represents a hydrogen atom and when the polymer of formula (I) contains only one set of repeating units; and

(b) at least one nucleic acid having improved transfection efficient and reduced cytotoxicity; a method for preparing a polyplex; a method for preparing the polymer of formula (I) for use in the polyplex; a method for transferring at least one nucleic acid into a cell and a pharmaceutical composition comprising the polyplex and an adjuvant; use of a polyplex or of a pharmaceutical composition according to the invention in transferring at least one nucleic acid into a cell; and use of a polymer of formula (I) as defined above in transferring of at least one nucleic acid into a cell..

To be accompanied when published by Figure 1 of the accompanying drawings.

SEQUENCE LISTING

<110>  Université de Liège

<120>  IMPROVEMENTS IN OR RELATING TO ORGANIC MATERIAL

<130>  P10076EP

<160>  2

<170>  PatentIn version 3.5

<210>  1
<211>  5130
<212>  DNA
<213>  Aequorea victoria

<220>
<221>  CDS
<222>  (728)..(1795)

<220>
<221>  exon
<222>  (728)..(1795)

<400>  1
tagttattaa tagtaatcaa ttacggggtc attagttcat agcccatata tggagttccg      60

cgttacataa cttacggtaa atggcccgcc tggctgaccg cccaacgacc cccgcccatt     120

gacgtcaata atgacgtatg ttcccatagt aacgccaata gggactttcc attgacgtca     180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggactcag atctcgagct ctctcgacgc aggactcggc ttgctgaagc gcgcacggca 660

agaggcgagg ggcggcgact ggtgagtacg ccaaaaattt tgactagcgg aggctagaag 720

gagagag atg ggt gcg aga gcg tca gta tta agc ggg gga gaa tta gat 769
Met Gly Ala Arg Ala Ser Val Leu Ser Gly Gly Glu Leu Asp
1               5                   10

cga tgg gaa aaa att cgg tta agg cca ggg gga aag aaa aaa tat aaa 817
Arg Trp Glu Lys Ile Arg Leu Arg Pro Gly Gly Lys Lys Lys Tyr Lys
15                  20                  25                  30

tta aaa cat ata gta tgg gca agc agg gag cta gaa cga ttc gca gtt 865
Leu Lys His Ile Val Trp Ala Ser Arg Glu Leu Glu Arg Phe Ala Val
                35                  40                  45

aat cct ggc ctg tta gaa aca tca gaa ggc tgt aga caa ata ctg gga 913
Asn Pro Gly Leu Leu Glu Thr Ser Glu Gly Cys Arg Gln Ile Leu Gly
            50                  55                  60

cag cta caa cca tcc ctt cag aca gga tca gaa gaa ctt aga tca tta 961
Gln Leu Gln Pro Ser Leu Gln Thr Gly Ser Glu Glu Leu Arg Ser Leu
        65                  70                  75

tat aat aca gta gca acc ctc tat tgt gtg cat caa agg ata gag ata 1009
Tyr Asn Thr Val Ala Thr Leu Tyr Cys Val His Gln Arg Ile Glu Ile
    80                  85                  90

aaa gac acc aag gaa gct tcg aat tct gca gtc gac ggt acc gcg ggc 1057
Lys Asp Thr Lys Glu Ala Ser Asn Ser Ala Val Asp Gly Thr Ala Gly
95                  100                 105                 110

ccg gga tcc atc gcc acc atg gtg agc aag ggc gag gag ctg ttc acc 1105
Pro Gly Ser Ile Ala Thr Met Val Ser Lys Gly Glu Glu Leu Phe Thr
                115                 120                 125

ggg gtg gtg ccc atc ctg gtc gag ctg gac ggc gac gta aac ggc cac 1153
Gly Val Val Pro Ile Leu Val Glu Leu Asp Gly Asp Val Asn Gly His
            130                 135                 140

aag ttc agc gtg tcc ggc gag ggc gag ggc gat gcc acc tac ggc aag 1201
Lys Phe Ser Val Ser Gly Glu Gly Glu Gly Asp Ala Thr Tyr Gly Lys
        145                 150                 155

ctg acc ctg aag ttc atc tgc acc acc ggc aag ctg ccc gtg ccc tgg 1249
Leu Thr Leu Lys Phe Ile Cys Thr Thr Gly Lys Leu Pro Val Pro Trp
    160                 165                 170

ccc acc ctc gtg acc acc ctg acc tac ggc gtg cag tgc ttc agc cgc 1297
Pro Thr Leu Val Thr Thr Leu Thr Tyr Gly Val Gln Cys Phe Ser Arg
175                 180                 185                 190

tac ccc gac cac atg aag cag cac gac ttc ttc aag tcc gcc atg ccc 1345
Tyr Pro Asp His Met Lys Gln His Asp Phe Phe Lys Ser Ala Met Pro
                195                 200                 205

gaa ggc tac gtc cag gag cgc acc atc ttc ttc aag gac gac ggc aac 1393
Glu Gly Tyr Val Gln Glu Arg Thr Ile Phe Phe Lys Asp Asp Gly Asn
            210                 215                 220

tac aag acc cgc gcc gag gtg aag ttc gag ggc gac acc ctg gtg aac 1441
Tyr Lys Thr Arg Ala Glu Val Lys Phe Glu Gly Asp Thr Leu Val Asn
        225                 230                 235

cgc atc gag ctg aag ggc atc gac ttc aag gag gac ggc aac atc ctg 1489
Arg Ile Glu Leu Lys Gly Ile Asp Phe Lys Glu Asp Gly Asn Ile Leu
    240                 245                 250

ggg cac aag ctg gag tac aac tac aac agc cac aac gtc tat atc atg 1537
Gly His Lys Leu Glu Tyr Asn Tyr Asn Ser His Asn Val Tyr Ile Met
255                 260                 265                 270

gcc gac aag cag aag aac ggc atc aag gtg aac ttc aag atc cgc cac 1585
Ala Asp Lys Gln Lys Asn Gly Ile Lys Val Asn Phe Lys Ile Arg His
                275                 280                 285

aac atc gag gac ggc agc gtg cag ctc gcc gac cac tac cag cag aac 1633
Asn Ile Glu Asp Gly Ser Val Gln Leu Ala Asp His Tyr Gln Gln Asn
            290                 295                 300

acc ccc atc ggc gac ggc ccc gtg ctg ctg ccc gac aac cac tac ctg 1681
Thr Pro Ile Gly Asp Gly Pro Val Leu Leu Pro Asp Asn His Tyr Leu
        305                 310                 315

agc acc cag tcc gcc ctg agc aaa gac ccc aac gag aag cgc gat cac 1729
Ser Thr Gln Ser Ala Leu Ser Lys Asp Pro Asn Glu Lys Arg Asp His
    320                 325                 330

atg gtc ctg ctg gag ttc gtg acc gcc gcc ggg atc act ctc ggc atg 1777
Met Val Leu Leu Glu Phe Val Thr Ala Ala Gly Ile Thr Leu Gly Met
335                 340                 345                 350

gac gag ctg tac aag taa agcggccgcg actctagatc ataatcagcc 1825
Asp Glu Leu Tyr Lys

                355

ataccacatt tgtagaggtt ttacttgctt taaaaaacct cccacacctc cccctgaacc 1885

tgaaacataa aatgaatgca attgttgttg ttaacttgtt tattgcagct tataatggtt 1945

acaaataaag caatagcatc acaaatttca caaataaagc atttttttca ctgcattcta 2005

gttgtggttt gtccaaactc atcaatgtat cttaaggcgt aaattgtaag cgttaatatt 2065

ttgttaaaat tcgcgttaaa tttttgttaa atcagctcat tttttaacca ataggccgaa 2125

atcggcaaaa tcccttataa atcaaaagaa tagaccgaga tagggttgag tgttgttcca 2185

gtttggaaca agagtccact attaaagaac gtggactcca acgtcaaagg gcgaaaaacc 2245

gtctatcagg gcgatggccc actacgtgaa ccatcaccct aatcaagttt tttggggtcg 2305

aggtgccgta aagcactaaa tcggaaccct aaagggagcc cccgatttag agcttgacgg 2365

ggaaagccgg cgaacgtggc gagaaaggaa gggaagaaag cgaaaggagc gggcgctagg 2425

gcgctggcaa gtgtagcggt cacgctgcgc gtaaccacca cacccgccgc gcttaatgcg 2485

ccgctacagg gcgcgtcagg tggcactttt cggggaaatg tgcgcggaac ccctatttgt 2545

ttatttttct aaatacattc aaatatgtat ccgctcatga gacaataacc ctgataaatg 2605

cttcaataat attgaaaaag gaagagtcct gaggcggaaa gaaccagctg tggaatgtgt 2665

gtcagttagg gtgtggaaag tccccaggct ccccagcagg cagaagtatg caaagcatgc 2725

atctcaatta gtcagcaacc aggtgtggaa agtccccagg ctccccagca ggcagaagta 2785

tgcaaagcat gcatctcaat tagtcagcaa ccatagtccc gcccctaact ccgcccatcc 2845

cgcccctaac tccgcccagt tccgcccatt ctccgcccca tggctgacta atttttttta 2905

tttatgcaga ggccgaggcc gcctcggcct ctgagctatt ccagaagtag tgaggaggct 2965

tttttggagg cctaggcttt tgcaaagatc gatcaagaga caggatgagg atcgtttcgc 3025

atgattgaac aagatggatt gcacgcaggt tctccggccg cttgggtgga gaggctattc 3085

ggctatgact gggcacaaca gacaatcggc tgctctgatg ccgccgtgtt ccggctgtca 3145

gcgcaggggc gcccggttct ttttgtcaag accgacctgt ccggtgccct gaatgaactg 3205

caagacgagg cagcgcggct atcgtggctg gccacgacgg gcgttccttg cgcagctgtg 3265

ctcgacgttg tcactgaagc gggaagggac tggctgctat tgggcgaagt gccggggcag 3325

gatctcctgt catctcacct tgctcctgcc gagaaagtat ccatcatggc tgatgcaatg 3385

cggcggctgc atacgcttga tccggctacc tgcccattcg accaccaagc gaaacatcgc 3445

atcgagcgag cacgtactcg gatggaagcc ggtcttgtcg atcaggatga tctggacgaa 3505

gagcatcagg ggctcgcgcc agccgaactg ttcgccaggc tcaaggcgag catgcccgac 3565

ggcgaggatc tcgtcgtgac ccatggcgat gcctgcttgc cgaatatcat ggtggaaaat 3625

ggccgctttt ctggattcat cgactgtggc cggctgggtg tggcggaccg ctatcaggac 3685

atagcgttgg ctacccgtga tattgctgaa gagcttggcg gcgaatgggc tgaccgcttc 3745

ctcgtgcttt acggtatcgc cgctcccgat tcgcagcgca tcgccttcta tcgccttctt 3805

gacgagttct tctgagcggg actctggggt tcgaaatgac cgaccaagcg acgcccaacc 3865

tgccatcacg agatttcgat tccaccgccg ccttctatga aaggttgggc ttcggaatcg 3925

ttttccggga cgccggctgg atgatcctcc agcgcgggga tctcatgctg gagttcttcg 3985

cccaccctag ggggaggcta actgaaacac ggaaggagac aataccggaa ggaacccgcg 4045

ctatgacggc aataaaaaga cagaataaaa cgcacggtgt tgggtcgttt gttcataaac 4105

gcggggttcg gtcccagggc tggcactctg tcgatacccc accgagaccc cattggggcc 4165

aatacgcccg cgtttcttcc ttttccccac cccacccccc aagttcgggt gaaggcccag 4225

ggctcgcagc caacgtcggg gcggcaggcc ctgccatagc ctcaggttac tcatatatac 4285

tttagattga tttaaaactt catttttaat ttaaaaggat ctaggtgaag atcctttttg 4345

ataatctcat gaccaaaatc ccttaacgtg agttttcgtt ccactgagcg tcagaccccg 4405

tagaaaagat caaaggatct tcttgagatc ctttttttct gcgcgtaatc tgctgcttgc 4465

aaacaaaaaa accaccgcta ccagcggtgg tttgtttgcc ggatcaagag ctaccaactc 4525

tttttccgaa ggtaactggc ttcagcagag cgcagatacc aaatactgtc cttctagtgt 4585

agccgtagtt aggccaccac ttcaagaact ctgtagcacc gcctacatac ctcgctctgc 4645

taatcctgtt accagtggct gctgccagtg gcgataagtc gtgtcttacc gggttggact 4705

caagacgata gttaccggat aaggcgcagc ggtcgggctg aacggggggt tcgtgcacac 4765

agcccagctt ggagcgaacg acctacaccg aactgagata cctacagcgt gagctatgag 4825

aaagcgccac gcttcccgaa gggagaaagg cggacaggta tccggtaagc ggcagggtcg 4885

gaacaggaga gcgcacgagg gagcttccag ggggaaacgc ctggtatctt tatagtcctg 4945

tcgggtttcg ccacctctga cttgagcgtc gatttttgtg atgctcgtca ggggggcgga 5005

gcctatggaa aaacgccagc aacgcggcct ttttacggtt cctggccttt tgctggcctt     5065

ttgctcacat gttctttcct gcgttatccc ctgattctgt ggataaccgt attaccgcca     5125

tgcat                                                                 5130

<210>  2
<211>  355
<212>  PRT
<213>  Aequorea victoria

<400>  2

Met Gly Ala Arg Ala Ser Val Leu Ser Gly Gly Glu Leu Asp Arg Trp
1               5                   10                  15

Glu Lys Ile Arg Leu Arg Pro Gly Gly Lys Lys Lys Tyr Lys Leu Lys
            20                  25                  30

His Ile Val Trp Ala Ser Arg Glu Leu Glu Arg Phe Ala Val Asn Pro
        35                  40                  45

Gly Leu Leu Glu Thr Ser Glu Gly Cys Arg Gln Ile Leu Gly Gln Leu
    50                  55                  60

Gln Pro Ser Leu Gln Thr Gly Ser Glu Glu Leu Arg Ser Leu Tyr Asn
65                  70                  75                  80

Thr Val Ala Thr Leu Tyr Cys Val His Gln Arg Ile Glu Ile Lys Asp
                85                  90                  95

Thr Lys Glu Ala Ser Asn Ser Ala Val Asp Gly Thr Ala Gly Pro Gly
            100                 105                 110

Ser Ile Ala Thr Met Val Ser Lys Gly Glu Glu Leu Phe Thr Gly Val
        115                 120                 125

Val Pro Ile Leu Val Glu Leu Asp Gly Asp Val Asn Gly His Lys Phe
    130                 135                 140

Ser Val Ser Gly Glu Gly Glu Gly Asp Ala Thr Tyr Gly Lys Leu Thr
145                 150                 155                 160

Leu Lys Phe Ile Cys Thr Thr Gly Lys Leu Pro Val Pro Trp Pro Thr
                165                 170                 175

Leu Val Thr Thr Leu Thr Tyr Gly Val Gln Cys Phe Ser Arg Tyr Pro
            180                 185                 190

Asp His Met Lys Gln His Asp Phe Phe Lys Ser Ala Met Pro Glu Gly
        195                 200                 205

Tyr Val Gln Glu Arg Thr Ile Phe Phe Lys Asp Asp Gly Asn Tyr Lys
    210                 215                 220

Thr Arg Ala Glu Val Lys Phe Glu Gly Asp Thr Leu Val Asn Arg Ile
225                 230                 235                 240

Glu Leu Lys Gly Ile Asp Phe Lys Glu Asp Gly Asn Ile Leu Gly His
                245                 250                 255

Lys Leu Glu Tyr Asn Tyr Asn Ser His Asn Val Tyr Ile Met Ala Asp
            260                 265                 270

Lys Gln Lys Asn Gly Ile Lys Val Asn Phe Lys Ile Arg His Asn Ile
        275                 280                 285

Glu Asp Gly Ser Val Gln Leu Ala Asp His Tyr Gln Gln Asn Thr Pro
    290                 295                 300

Ile Gly Asp Gly Pro Val Leu Leu Pro Asp Asn His Tyr Leu Ser Thr
305                 310                 315                 320

Gln Ser Ala Leu Ser Lys Asp Pro Asn Glu Lys Arg Asp His Met Val
                325                 330                 335

Leu Leu Glu Phe Val Thr Ala Ala Gly Ile Thr Leu Gly Met Asp Glu
            340                 345                 350

Leu Tyr Lys
        355

<210> 3
<211> 9681

<212>  DNA
<213>  Artificial Sequence

<220>
<223>  Plasmid sequence


<220>
<221>  CDS
<222>  (5009)..(5692)

<400>  1
ttctcatgtt tgacagctta tcatcgcaga tccgggcaac gttgttgcca ttgctgcagg      60

cgcagaactg gtaggtatgg aagatctata cattgaatca atattggcaa ttagccatat     120

tagtcattgg ttatatagca taaatcaata ttggctattg gccattgcat acgttgtatc     180

tatatcataa tatgtacatt tatattggct catgtccaat atgaccgcca tgttgacatt     240

gattattgac tagttattaa tagtaatcaa ttacggggtc attagttcat agcccatata     300

tggagttccg cgttacataa cttacggtaa atggcccgcc tggctgaccg cccaacgacc     360

cccgcccatt gacgtcaata atgacgtatg ttcccatagt aacgccaata gggactttcc     420

attgacgtca atgggtggag tatttacggt aaactgccca cttggcagta catcaagtgt     480

atcatatgcc aagtccgccc cctattgacg tcaatgacgg taaatggccc gcctggcatt     540

atgcccagta catgacctta cgggactttc ctacttggca gtacatctac gtattagtca     600

tcgctattac catggtgatg cggttttggc agtacaccaa tgggcgtgga tagcggtttg     660

actcacgggg atttccaagt ctccacccca ttgacgtcaa tgggagtttg ttttggcacc     720

aaaatcaacg ggactttcca aaatgtcgta ataaccccgc cccgttgacg caaatgggcg     780

gtaggcgtgt acggtgggag gtctatataa gcagagctcg tttagtgaac cgtcagatct     840

ctagaagctg gctgggccgg cttggcctcc attgagatcc ggaggtgagc tggcggacgc     900

tggggaccgg cgggacagcg ggtctcgcgc tccgcctggg ctcacgaggc ggctgcgaac     960

cgaggggacc gcgaggacac gtccttgagg cccggcggca gggcggcggc cagggaaccc    1020

gtgggccggg tgggcctgag ggccagtgca gcgacggggc gtccgtgggt tcagtccgga    1080

gggcgctgtg ctgacccggc ggtcacggtc atccgtgtac gagtgcctgg cagaggggac    1140

agcgggagcc ctagagaaca agggttcacg cccaggcgct gccttgcggc aggacccgtc    1200

tttaaagtga tggagatgta ggctgcaaat tgacccgatc cgcgtgggta cggcaggttg    1260

tgctcttcct agtgggcccg gaggaggatg cgaggtctgt acaggcacag ctagccctcc 1320

gaagtcggtc tctcgacctt tatgttatag tctttctcct aaggggcgta gtctcattga 1380

tactgatgtc aatttacctt atgcacctat tggaattatt tggtttgaat gaagaaaatg 1440

gccttagagg ttcgcaagta aaacagtaga cttctgaggt tgggcgaagt taatagtctt 1500

tcatcgaaag cggacagatg gaaatctttc ctctcttgag gttgtatctt ttcgggctaa 1560

ggcctgtaaa ggcacaggaa acgtctgtta ctggctcttt acttacaaag tcgttgtgta 1620

caattaaagc atcacacgaa gtagggaata cattttgttt ggttacttgt gatttatttg 1680

tattttattt atcgtagggc aaaagtcttg gatctacatt gggctgcgtt ttgtgggctt 1740

acttggtcgt agtgcctttc tagaacttga ccaggcaaga taaagggtgt gcttgctcac 1800

actgctatat ttttgtctaa tttgtagact gtgagctata ttctgggatc agtttctgaa 1860

tccttaaatc tatcttgaaa ttgtgctttt tttttttttt ttaagttctt aaaatagtag 1920

ttctttatgg ttccacttaa tactttggtc agagaacttg accatgaaga ggagatggac 1980

ttagaaactg gtaatgtatg gtaaagttgg gtacagaact tgttttctgt gcaaggtgat 2040

gatggccccc tttgatggat gggtaggtta agaatgctga gtgttcatcc tgttgggatt 2100

agacgtggct acagtgtagg attagggggt cttaccctca aagggacaac ctgcacatat 2160

cagtgtagat attttctaat caaagagtgg gttgtgtact accggaagaa tgaatggact 2220

ggaagaatct cagagaagcc tgtctcttgg cagtggttgg tggttctatt cctagataga 2280

attagaattt aagtctggtg agtttaaatt ttcagccatt tgaggagtct gtgaaatgtt 2340

tgctgtatac tgtatttgga taagtaggca agttctttta ttgcagcgca gcagaaaagg 2400

gaccttccga gctttcataa actgctacac cggtgtcact ttgcctagtc acagagaaat 2460

gggaggtgct cctgagagac aagtttataa ttacggttac ttttgacata cctttgtaac 2520

actgctcagt ttgtagttcc aggtaggtgt ggttctctgt gcttggttgg attgtggaac 2580

aggattttgt gtaatttttt cccctcagat tatttaataa taataaaaaa agacaatgta 2640

cgtgcaggga agagtggcaa gattgccagg ccccgggaac cgagcagggt gggaggtctg 2700

gtgtgcctgg ggtagtgtgg gtatggggtt actagttggc catcacattg gggctcctca 2760

tgatagcaat ttacattttt ctcagcggaa tcacttttaa tagtgatgaa taaaattgac 2820

tcgttaagga agactattga agctttgtgg tgaagggtgg aggactggaa gtgctttaga 2880

tttacatttg aaactaattt accttctgaa accttgaagc cagtcttttc caacagtttg 2940

ctttagtttc tgattttaag agattctgtg agagaggcca gtggacatgt ggtttctcat 3000

gagaatccta gacaggttta aagtgtttgt tctttaaacc caggccctcc ataaaagtac 3060

tgtgtggttg tcctttcttg ggtatttgtg tacattagtg caagatgccg gagaggaaag 3120

tggtaaaact cattatgcag tactgaaaaa aagataaata ctgatgcctt ccacatctta 3180

tgccatacta gttgttcttt ccaaaattaa gacccacagg aagaaatata gtgagaatgt 3240

tgtgaatgat ttcatgacgc atgagtttac atggtactta cccgtgttcc ctgccttacc 3300

tgtcaactac ttttatgttg ctttctattt tttagttaat taaaactgtt atcagcaaca 3360

ttgattttta tgattgaatt aatgtgttgc tctgactatc ttgaaaacca gttatattgg 3420

cgtgtagcct ggtatattga aatatagagg aaaaatggaa tgttggaatc agagatgatg 3480

ttggctaatt ggcttcattc ttaaaagcca agcccccacc cttcttaccc cccaccctcg 3540

ccctagctac cacccttctt acctcccacc cttgccccag ctcccaccgt tcttaccccc 3600

accctcgccc cagtactttt ttgcctcctt taatttggtg gtggtggtcc cttttttttt 3660

gttttgtttt gtttttttcc agacagggtt tctctgtgta gcccagactg gctttgagct 3720

cagagatctg actgcctctg cctcccttgt caccactgcc tggctagttg gtgtcatcag 3780

tcaataagtc actttcctag tagatgtgtg agatctattt gaattcttgc tgacaatcag 3840

tgggctgtta gttggtttct tgtcatcatt tgtattctat tgtgtgtgtt tgtgtgcact 3900

ggtgtgtact gtggcctgag cgtggcggtc agagctcagt ctgcagtgtt agaagcagtg 3960

aggcttagat gcaagtgcat tatccacagg cccatctccc ggggtctagt ttttgtgact 4020

gattgttatg ggtaatagtt ttcattttgc tctatagttt taccatgaga aatatctctg 4080

taggccatca gactataaga ttacatttca aataggaatg tcaaacttgt taattagcca 4140

tcagatgtgg gaagggtggc agtcatttca ttagctcaga tcttgctatt tattctgttg 4200

aatcctcttg tcagacaaga agtcgtagtt ttgggggttt tttgaacaga gggccctgca 4260

atgagctgta tcttcactcc ttgggggtct ttctgtatga tacatacatg tttcatttta 4320

cccagcatct acttctactg actttccagt cttggctggc tgtaaaagtt taggagcctt 4380

ctacatctaa cacatttaaa tgggccttct tgcccctgta ctttgccagg agaaaagcca 4440

tcctagtgaa atgcatgccc ccgttttcct aatgtggaag tatagatcag aaaagatttc 4500

tggtgacagc caccttaggg aagttcctta tacattgtgc tagtgacatt ctgccttatt 4560

ctagagactg aaatgaaaag caggatttat ggcaaggtgc tcgagaagaa ggttctttgg 4620

tgttgccttt attaggcatg tggtggtgaa gaggatgctc gtgggctttc tgtttaacta 4680

aaaagtggga atagtctcat gtctgctttg tgctttattt attctgctcc tccttggctc 4740

acataactac aaggagttac tcttgaaact taacttaaag gaacatttgc atataagttg 4800

tttaccagtg agtaataagg gatttaggtg gttgaagaag tggcaagcac cgtgttaata 4860

gagcattaca ctcactttcc aggattatgc agaacgctgg aatagtcctg tacatgagat 4920

gctgcactta gggctgaaag tgagccccat gcatgctaag tgttcttcat atgtatatcc 4980

ttccaggatc tggatccagc ttttcaaa atg gaa gac gcc aaa aac ata aag 5032
                               Met Glu Asp Ala Lys Asn Ile Lys
                               1               5

aaa ggc ccg gcg cca ttc tat cct cta gag gat gga acc gct gga gag 5080
Lys Gly Pro Ala Pro Phe Tyr Pro Leu Glu Asp Gly Thr Ala Gly Glu
    10                  15                  20

caa ctg cat aag gct atg aag aga tac gcc ctg gtt cct gga aca att 5128
Gln Leu His Lys Ala Met Lys Arg Tyr Ala Leu Val Pro Gly Thr Ile
25                  30                  35                  40

gct ttt aca gat gca cat atc gag gtg aac atc atg tac gcg gaa tac 5176
Ala Phe Thr Asp Ala His Ile Glu Val Asn Ile Met Tyr Ala Glu Tyr
                45                  50                  55

ttc gaa atg tcc gtt cgg ttg gca gaa gct atg aaa cga tat ggg ctg 5224
Phe Glu Met Ser Val Arg Leu Ala Glu Ala Met Lys Arg Tyr Gly Leu
            60                  65                  70

aat aca aat cac aga atc gtc gta tgc agt gaa aac tct ctt caa ttc 5272
Asn Thr Asn His Arg Ile Val Val Cys Ser Glu Asn Ser Leu Gln Phe
        75                  80                  85

ttt atg ccg gtg ttg ggc gcg tta ttt atc gga gtt gca gtt gcg ccc 5320
Phe Met Pro Val Leu Gly Ala Leu Phe Ile Gly Val Ala Val Ala Pro
    90                  95                  100

gcg aac gac att tat aat gaa cgt gaa ttg ctc aac agt atg aac att 5368
Ala Asn Asp Ile Tyr Asn Glu Arg Glu Leu Leu Asn Ser Met Asn Ile
105                 110                 115                 120

tcg cag cct acc gta gtg ttt gtt tcc aaa aag ggg ttg caa aaa att 5416
Ser Gln Pro Thr Val Val Phe Val Ser Lys Lys Gly Leu Gln Lys Ile
                125                 130                 135

ttg aac gtg caa aaa aaa tta cca ata atc cag aaa att att atc atg 5464
Leu Asn Val Gln Lys Lys Leu Pro Ile Ile Gln Lys Ile Ile Ile Met

140                 145                 150

gat tct aaa acg gat tac cag gga ttt cag tcg atg tac acg ttc gtc        5512
Asp Ser Lys Thr Asp Tyr Gln Gly Phe Gln Ser Met Tyr Thr Phe Val
        155                 160                 165

aca tct cat cta cct ccc ggt ttt aat gaa tac gat ttt gta cca gag        5560
Thr Ser His Leu Pro Pro Gly Phe Asn Glu Tyr Asp Phe Val Pro Glu
    170                 175                 180

tcc ttt gat cgt gac aaa aca att gca ctg ata atg aat tcc tct gga        5608
Ser Phe Asp Arg Asp Lys Thr Ile Ala Leu Ile Met Asn Ser Ser Gly
185                 190                 195                 200

tct act ggg tta cct aag ggt gtg gcc ctt ccg cat aga act gcc tgc        5656
Ser Thr Gly Leu Pro Lys Gly Val Ala Leu Pro His Arg Thr Ala Cys
                205                 210                 215

gtc aga ttc tcg cat gcc aga gat cct att ttt ggc aatcaaatca             5702
Val Arg Phe Ser His Ala Arg Asp Pro Ile Phe Gly
            220                 225

ttccggatac tgcgatttta agtgttgttc cattccatca cggttttgga atgtttacta      5762

cactcggata tttgatatgt ggatttcgag tcgtcttaat gtatagattt gaagaagagc      5822

tgtttttacg atcccttcag gattacaaaa ttcaaagtgc gttgctagta ccaaccctat      5882

tttcattctt cgccaaaagc actctgattg acaaatacga tttatctaat ttacacgaaa      5942

ttgcttctgg gggcgcacct ctttcgaaag aagtcgggga agcggttgca aaacgcttcc      6002

atcttccagg gatacgacaa ggatatgggc tcactgagac tacatcagct attctgatta      6062

cacccgaggg ggatgataaa ccgggcgcgg tcggtaaagt tgttccattt tttgaagcga      6122

aggttgtgga tctggatacc gggaaaacgc tgggcgttaa tcagagaggc gaattatgtg      6182

tcagaggacc tatgattatg tccggttatg taaacaatcc ggaagcgacc aacgccttga      6242

ttgacaagga tggatggcta cattctggag acatagctta ctgggacgaa gacgaacact      6302

tcttcatagt tgaccgcttg aagtctttaa ttaaatacaa aggatatcag gtggcccccg      6362

ctgaattgga atcgatattg ttacaacacc ccaacatctt cgacgcgggc gtggcaggtc      6422

ttcccgacga tgacgccggt gaacttcccg ccgccgttgt tgttttggag cacggaaaga      6482

cgatgacgga aaaagagatc gtggattacg tcgccagtca agtaacaacc gcgaaaaagt      6542

tgcgcggagg agttgtgttt gtggacgaag taccgaaagg tcttaccgga aaactcgacg      6602

caagaaaaat cagagagatc ctcataaagg ccaagaaggg cggaaagtcc aaattgtaaa      6662

agaattcttt ctgctaggat ccagacatga taagatacat tgatgagttt ggacaaacca 6722

caactagaat gcagtgaaaa aaatgcttta tttgtgaaat ttgtgatgct attgctttat 6782

ttgtaaccat tataagctgc aataaacaag ttaacaacaa caattgcatt cattttatgt 6842

ttcaggttca gggggaggtg ggaggttttt taaagcaagt aaaacctcta caaatgtgg 6902

tatggctgat tatgatccgg ctgcctcgcg cgtttcggtg atgacggtga aaacctctga 6962

cacatgcagc tcccggagac ggtcacagct tgtctgtaag cggatgccgg gagcagacaa 7022

gcccgtcagg gcgcgtcagc gggtgttggc gggtgtcggg gcgcagccat gacgtcgact 7082

ctaccggaaa tacaggaacg cacgctggat ggcccttcgc tgggatggtg aaaccatgaa 7142

aaatggcagc ttcagtggat taagtggggg taatgtggcc tgtaccctct ggttgcatag 7202

gtattcatac ggttaaaatt tatcaggcgc gatcgcggca gtttttcggg tggtttgttg 7262

ccatttttac ctgtctgctg ccgtgatcgc gctgaacgcg ttttagcggt gcgtacaatt 7322

aagggattat ggtaaatcca cttactgtct gccctcgtag ccatcgtaga gcggccctag 7382

ctacgtagat gataagctgt caaacatgag aattcttgaa gacgaaaggg cctcgtgata 7442

cgcctatttt tataggttaa tgtcatgata ataatggttt cttagacgtc aggtggcact 7502

tttcggggaa atgtgcgcgg aacccctatt tgtttatttt tctaaataca ttcaaatatg 7562

tatccgctca tgagacaata accctgataa atgcttcaat aatattgaaa aaggaagagt 7622

atgagccata ttcaacggga aacgtcttgc tcgaggccgc gattaaattc caacatggat 7682

gctgatttat atgggtataa atgggctcgc gataatgtcg ggcaatcagg tgcgacaatc 7742

tatcgattgt atgggaagcc cgatgcgcca gagttgtttc tgaaacatgg caaaggtagc 7802

gttgccaatg atgttacaga tgagatggtc agactaaact ggctgacgga atttatgcct 7862

cttccgacca tcaagcattt tatccgtact cctgatgatg catggttact caccactgcg 7922

atccccggga aaacagcatt ccaggtatta gaagaatatc ctgattcagg tgaaaatatt 7982

gttgatgcgc tggcagtgtt cctgcgccgg ttgcattcga ttcctgtttg taattgtcct 8042

tttaacagcg atcgcgtatt tcgtctcgct caggcgcaat cacgaatgaa taacggtttg 8102

gttgatgcga gtgattttga tgacgagcgt aatggctggc ctgttgaaca agtctggaaa 8162

gaaatgcata agcttttgcc attctcaccg gattcagtcg tcactcatgg tgatttctca 8222

cttgataacc ttatttttga cgaggggaaa ttaataggtt gtattgatgt tggacgagtc 8282

ggaatcgcag accgatacca ggatcttgcc atcctatgga actgcctcgg tgagttttct 8342

ccttcattac agaaacggct ttttcaaaaa tatggtattg ataatcctga tatgaataaa 8402

ttgcagtttc atttgatgct cgatgagttt ttctaatcag aattggttaa ttggttgtaa 8462

cactggcaga gcattacgct gacttgacgg gacggcggct ttgttgaata aatcgaactt 8522

ttgctgagtt gaaggatcag atcacgcatc ttcccgacaa cgcagaccgt tccgtggcaa 8582

agcaaaagtt caaaatcacc aactggtcca cctacaacaa agctctcatc aaccgtggct 8642

ccctcacttt ctggctggat gatggggcga ttcaggcctg gtatgagtca gcaacacctt 8702

cttcacgagg cagagatctc ccttaacgtg agttttcgtt ccactgagcg tcagaccccg 8762

tagaaaagat caaaggatct tcttgagatc ctttttttct gcgcgtaatc tgctgcttgc 8822

aaacaaaaaa accaccgcta ccagcggtgg tttgtttgcc ggatcaagag ctaccaactc 8882

tttttccgaa ggtaactggc ttcagcagag cgcagatacc aaatactgtc cttctagtgt 8942

agccgtagtt aggccaccac ttcaagaact ctgtagcacc gcctacatac ctcgctctgc 9002

taatcctgtt accagtggct gctgccagtg gcgataagtc gtgtcttacc gggttggact 9062

caagacgata gttaccggat aaggcgcagc ggtcgggctg aacggggggt tcgtgcacac 9122

agcccagctt ggagcgaacg acctacaccg aactgagata cctacagcgt gagctatgag 9182

aaagcgccac gcttcccgaa gggagaaagg cggacaggta tccggtaagc ggcagggtcg 9242

gaacaggaga gcgcacgagg gagcttccag ggggaaacgc ctggtatctt tatagtcctg 9302

tcgggtttcg ccacctctga cttgagcgtc gatttttgtg atgctcgtca ggggggcgga 9362

gcctatggaa aaacgccagc aacgcggcct ttttacggtt cctggccttt tgctggcctt 9422

gaagctgtcc ctgatggtcg tcatctacct gcctggacag catggcctgc aacgcgggca 9482

tcccgatgcc gccggaagcg agaagaatca taatggggaa ggccatccag cctcgcgtcg 9542

cgaacgccag caagacgtag cccagcgcgt cggccccgag atgcgccgcg tgcggctgct 9602

ggagatggcg gacgcgatgg atatgttctg ccaagggttg gtttgcgcaa gaagagcggc 9662

ctgtgtggcc gtcgaccaa 9681

<210> 4
<211> 228
<212> PRT
<213> Artificial Sequence

<220>
<223>  Synthetic Construct

<400>  2

Met Glu Asp Ala Lys Asn Ile Lys Lys Gly Pro Ala Pro Phe Tyr Pro
1               5                   10                  15

Leu Glu Asp Gly Thr Ala Gly Glu Gln Leu His Lys Ala Met Lys Arg
            20                  25                  30

Tyr Ala Leu Val Pro Gly Thr Ile Ala Phe Thr Asp Ala His Ile Glu
        35                  40                  45

Val Asn Ile Met Tyr Ala Glu Tyr Phe Glu Met Ser Val Arg Leu Ala
    50                  55                  60

Glu Ala Met Lys Arg Tyr Gly Leu Asn Thr Asn His Arg Ile Val Val
65                  70                  75                  80

Cys Ser Glu Asn Ser Leu Gln Phe Phe Met Pro Val Leu Gly Ala Leu
                85                  90                  95

Phe Ile Gly Val Ala Val Ala Pro Ala Asn Asp Ile Tyr Asn Glu Arg
            100                 105                 110

Glu Leu Leu Asn Ser Met Asn Ile Ser Gln Pro Thr Val Val Phe Val
        115                 120                 125

Ser Lys Lys Gly Leu Gln Lys Ile Leu Asn Val Gln Lys Lys Leu Pro
    130                 135                 140

Ile Ile Gln Lys Ile Ile Ile Met Asp Ser Lys Thr Asp Tyr Gln Gly
145                 150                 155                 160

Phe Gln Ser Met Tyr Thr Phe Val Thr Ser His Leu Pro Pro Gly Phe
                165                 170                 175

Asn Glu Tyr Asp Phe Val Pro Glu Ser Phe Asp Arg Asp Lys Thr Ile
            180                 185                 190

Ala Leu Ile Met Asn Ser Ser Gly Ser Thr Gly Leu Pro Lys Gly Val

195                 200                 205

Ala Leu Pro His Arg Thr Ala Cys Val Arg Phe Ser His Ala Arg Asp
    210                 215                 220

Pro Ile Phe Gly
225

SEQUENCE LISTING

<110>  Université de Liège
Université Pierre et Marie Curie (UPMC)
Centre National de la Recherche Scientifique (CNRS)

<120>  IMPROVEMENTS IN OR RELATING TO ORGANIC MATERIAL

<130>  P10076EP

<160>  2

<170>  PatentIn version 3.5

<210>  1
<211>  5130
<212>  DNA
<213>  Aequorea victoria


<220>
<221>  CDS
<222>  (728)..(1795)

<220>
<221>  exon
<222>  (728)..(1795)

<400>  1
tagttattaa tagtaatcaa ttacggggtc attagttcat agcccatata tggagttccg      60

cgttacataa cttacggtaa atggcccgcc tggctgaccg cccaacgacc cccgcccatt     120

gacgtcaata atgacgtatg ttcccatagt aacgccaata gggactttcc attgacgtca     180

atgggtggag tatttacggt aaactgccca cttggcagta catcaagtgt atcatatgcc     240

aagtacgccc cctattgacg tcaatgacgg taaatggccc gcctggcatt atgcccagta     300

catgacctta tgggactttc ctacttggca gtacatctac gtattagtca tcgctattac     360

catggtgatg cggttttggc agtacatcaa tgggcgtgga tagcggtttg actcacgggg     420

atttccaagt ctccacccca ttgacgtcaa tgggagtttg ttttggcacc aaaatcaacg     480

ggactttcca aaatgtcgta acaactccgc cccattgacg caaatgggcg gtaggcgtgt     540

acggtgggag gtctatataa gcagagctgg tttagtgaac cgtcagatcc gctagcgcta     600

ccggactcag atctcgagct ctctcgacgc aggactcggc ttgctgaagc gcgcacggca     660

agaggcgagg ggcggcgact ggtgagtacg ccaaaaattt tgactagcgg aggctagaag     720

gagagag atg ggt gcg aga gcg tca gta tta agc ggg gga gaa tta gat      769
        Met Gly Ala Arg Ala Ser Val Leu Ser Gly Gly Glu Leu Asp
        1               5                   10

cga tgg gaa aaa att cgg tta agg cca ggg gga aag aaa aaa tat aaa      817
Arg Trp Glu Lys Ile Arg Leu Arg Pro Gly Gly Lys Lys Lys Tyr Lys
15                  20                  25                  30

tta aaa cat ata gta tgg gca agc agg gag cta gaa cga ttc gca gtt      865
Leu Lys His Ile Val Trp Ala Ser Arg Glu Leu Glu Arg Phe Ala Val

                35                  40                  45

aat cct ggc ctg tta gaa aca tca gaa ggc tgt aga caa ata ctg gga 913
Asn Pro Gly Leu Leu Glu Thr Ser Glu Gly Cys Arg Gln Ile Leu Gly
            50                  55                  60

cag cta caa cca tcc ctt cag aca gga tca gaa gaa ctt aga tca tta 961
Gln Leu Gln Pro Ser Leu Gln Thr Gly Ser Glu Glu Leu Arg Ser Leu
        65                  70                  75

tat aat aca gta gca acc ctc tat tgt gtg cat caa agg ata gag ata 1009
Tyr Asn Thr Val Ala Thr Leu Tyr Cys Val His Gln Arg Ile Glu Ile
    80                  85                  90

aaa gac acc aag gaa gct tcg aat tct gca gtc gac ggt acc gcg ggc 1057
Lys Asp Thr Lys Glu Ala Ser Asn Ser Ala Val Asp Gly Thr Ala Gly
95                  100                 105                 110

ccg gga tcc atc gcc acc atg gtg agc aag ggc gag gag ctg ttc acc 1105
Pro Gly Ser Ile Ala Thr Met Val Ser Lys Gly Glu Glu Leu Phe Thr
                115                 120                 125

ggg gtg gtg ccc atc ctg gtc gag ctg gac ggc gac gta aac ggc cac 1153
Gly Val Val Pro Ile Leu Val Glu Leu Asp Gly Asp Val Asn Gly His
            130                 135                 140

aag ttc agc gtg tcc ggc gag ggc gag ggc gat gcc acc tac ggc aag 1201
Lys Phe Ser Val Ser Gly Glu Gly Glu Gly Asp Ala Thr Tyr Gly Lys
        145                 150                 155

ctg acc ctg aag ttc atc tgc acc acc ggc aag ctg ccc gtg ccc tgg 1249
Leu Thr Leu Lys Phe Ile Cys Thr Thr Gly Lys Leu Pro Val Pro Trp
    160                 165                 170

ccc acc ctc gtg acc acc ctg acc tac ggc gtg cag tgc ttc agc cgc 1297
Pro Thr Leu Val Thr Thr Leu Thr Tyr Gly Val Gln Cys Phe Ser Arg
175                 180                 185                 190

tac ccc gac cac atg aag cag cac gac ttc ttc aag tcc gcc atg ccc 1345
Tyr Pro Asp His Met Lys Gln His Asp Phe Phe Lys Ser Ala Met Pro
                195                 200                 205

gaa ggc tac gtc cag gag cgc acc atc ttc ttc aag gac gac ggc aac 1393
Glu Gly Tyr Val Gln Glu Arg Thr Ile Phe Phe Lys Asp Asp Gly Asn
            210                 215                 220

tac aag acc cgc gcc gag gtg aag ttc gag ggc gac acc ctg gtg aac 1441
Tyr Lys Thr Arg Ala Glu Val Lys Phe Glu Gly Asp Thr Leu Val Asn
        225                 230                 235

cgc atc gag ctg aag ggc atc gac ttc aag gag gac ggc aac atc ctg 1489
Arg Ile Glu Leu Lys Gly Ile Asp Phe Lys Glu Asp Gly Asn Ile Leu
    240                 245                 250

ggg cac aag ctg gag tac aac tac aac agc cac aac gtc tat atc atg 1537
Gly His Lys Leu Glu Tyr Asn Tyr Asn Ser His Asn Val Tyr Ile Met
255                 260                 265                 270

gcc gac aag cag aag aac ggc atc aag gtg aac ttc aag atc cgc cac 1585
Ala Asp Lys Gln Lys Asn Gly Ile Lys Val Asn Phe Lys Ile Arg His
                275                 280                 285

aac atc gag gac ggc agc gtg cag ctc gcc gac cac tac cag cag aac 1633

Asn Ile Glu Asp Gly Ser Val Gln Leu Ala Asp His Tyr Gln Gln Asn
            290                 295                 300

acc ccc atc ggc gac ggc ccc gtg ctg ctg ccc gac aac cac tac ctg      1681
Thr Pro Ile Gly Asp Gly Pro Val Leu Leu Pro Asp Asn His Tyr Leu
        305                 310                 315

agc acc cag tcc gcc ctg agc aaa gac ccc aac gag aag cgc gat cac      1729
Ser Thr Gln Ser Ala Leu Ser Lys Asp Pro Asn Glu Lys Arg Asp His
    320                 325                 330

atg gtc ctg ctg gag ttc gtg acc gcc gcc ggg atc act ctc ggc atg      1777
Met Val Leu Leu Glu Phe Val Thr Ala Ala Gly Ile Thr Leu Gly Met
335                 340                 345                 350

gac gag ctg tac aag taa agcggccgcg actctagatc ataatcagcc             1825
Asp Glu Leu Tyr Lys
                355

ataccacatt tgtagaggtt ttacttgctt taaaaaacct cccacacctc cccctgaacc    1885

tgaaacataa aatgaatgca attgttgttg ttaacttgtt tattgcagct tataatggtt    1945

acaaataaag caatagcatc acaaatttca caaataaagc atttttttca ctgcattcta    2005

gttgtggttt gtccaaactc atcaatgtat cttaaggcgt aaattgtaag cgttaatatt    2065

ttgttaaaat tcgcgttaaa tttttgttaa atcagctcat tttttaacca ataggccgaa    2125

atcggcaaaa tcccttataa atcaaaagaa tagaccgaga tagggttgag tgttgttcca    2185

gtttggaaca agagtccact attaaagaac gtggactcca acgtcaaagg gcgaaaaacc    2245

gtctatcagg gcgatggccc actacgtgaa ccatcaccct aatcaagttt tttggggtcg    2305

aggtgccgta aagcactaaa tcggaaccct aaagggagcc cccgatttag agcttgacgg    2365

ggaaagccgg cgaacgtggc gagaaaggaa gggaagaaag cgaaaggagc gggcgctagg    2425

gcgctggcaa gtgtagcggt cacgctgcgc gtaaccacca cacccgccgc gcttaatgcg    2485

ccgctacagg gcgcgtcagg tggcactttt cggggaaatg tgcgcggaac ccctatttgt    2545

ttatttttct aaatacattc aaatatgtat ccgctcatga gacaataacc ctgataaatg    2605

cttcaataat attgaaaaag gaagagtcct gaggcggaaa gaaccagctg tggaatgtgt    2665

gtcagttagg gtgtggaaag tccccaggct ccccagcagg cagaagtatg caaagcatgc    2725

atctcaatta gtcagcaacc aggtgtggaa agtccccagg ctccccagca ggcagaagta    2785

tgcaaagcat gcatctcaat tagtcagcaa ccatagtccc gcccctaact ccgcccatcc    2845

cgcccctaac tccgcccagt tccgcccatt ctccgcccca tggctgacta atttttttta    2905

tttatgcaga ggccgaggcc gcctcggcct ctgagctatt ccagaagtag tgaggaggct    2965

tttttggagg cctaggcttt tgcaaagatc gatcaagaga caggatgagg atcgtttcgc    3025

atgattgaac aagatggatt gcacgcaggt tctccggccg cttgggtgga gaggctattc    3085

ggctatgact gggcacaaca gacaatcggc tgctctgatg ccgccgtgtt ccggctgtca    3145

gcgcaggggc gcccggttct ttttgtcaag accgacctgt ccggtgccct gaatgaactg 3205

caagacgagg cagcgcggct atcgtggctg gccacgacgg gcgttccttg cgcagctgtg 3265

ctcgacgttg tcactgaagc gggaagggac tggctgctat tgggcgaagt gccggggcag 3325

gatctcctgt catctcacct tgctcctgcc gagaaagtat ccatcatggc tgatgcaatg 3385

cggcggctgc atacgcttga tccggctacc tgcccattcg accaccaagc gaaacatcgc 3445

atcgagcgag cacgtactcg gatggaagcc ggtcttgtcg atcaggatga tctggacgaa 3505

gagcatcagg ggctcgcgcc agccgaactg ttcgccaggc tcaaggcgag catgcccgac 3565

ggcgaggatc tcgtcgtgac ccatggcgat gcctgcttgc cgaatatcat ggtggaaaat 3625

ggccgctttt ctggattcat cgactgtggc cggctgggtg tggcggaccg ctatcaggac 3685

atagcgttgg ctacccgtga tattgctgaa gagcttggcg gcgaatgggc tgaccgcttc 3745

ctcgtgcttt acggtatcgc cgctcccgat tcgcagcgca tcgccttcta tcgccttctt 3805

gacgagttct tctgagcggg actctggggt tcgaaatgac cgaccaagcg acgcccaacc 3865

tgccatcacg agatttcgat tccaccgccg ccttctatga aaggttgggc ttcggaatcg 3925

ttttccggga cgccggctgg atgatcctcc agcgcgggga tctcatgctg gagttcttcg 3985

cccaccctag ggggaggcta actgaaacac ggaaggagac aataccggaa ggaacccgcg 4045

ctatgacggc aataaaaaga cagaataaaa cgcacggtgt tgggtcgttt gttcataaac 4105

gcggggttcg gtcccagggc tggcactctg tcgatacccc accgagaccc cattggggcc 4165

aatacgcccg cgtttcttcc ttttccccac cccacccccc aagttcgggt gaaggcccag 4225

ggctcgcagc caacgtcggg gcggcaggcc ctgccatagc ctcaggttac tcatatatac 4285

tttagattga tttaaaactt catttttaat ttaaaaggat ctaggtgaag atcctttttg 4345

ataatctcat gaccaaaatc ccttaacgtg agttttcgtt ccactgagcg tcagaccccg 4405

tagaaaagat caaaggatct tcttgagatc ctttttttct gcgcgtaatc tgctgcttgc 4465

aaacaaaaaa accaccgcta ccagcggtgg tttgtttgcc ggatcaagag ctaccaactc 4525

tttttccgaa ggtaactggc ttcagcagag cgcagatacc aaatactgtc cttctagtgt 4585

agccgtagtt aggccaccac ttcaagaact ctgtagcacc gcctacatac ctcgctctgc 4645

taatcctgtt accagtggct gctgccagtg gcgataagtc gtgtcttacc gggttggact 4705

caagacgata gttaccggat aaggcgcagc ggtcgggctg aacggggggt tcgtgcacac 4765

agcccagctt ggagcgaacg acctacaccg aactgagata cctacagcgt gagctatgag 4825

aaagcgccac gcttcccgaa gggagaaagg cggacaggta tccggtaagc ggcagggtcg 4885

gaacaggaga gcgcacgagg gagcttccag ggggaaacgc ctggtatctt tatagtcctg 4945

tcgggtttcg ccacctctga cttgagcgtc gatttttgtg atgctcgtca ggggggcgga 5005

gcctatggaa aaacgccagc aacgcggcct ttttacggtt cctggccttt tgctggcctt 5065

ttgctcacat gttctttcct gcgttatccc ctgattctgt ggataaccgt attaccgcca     5125

tgcat                                                                 5130

<210>  2
<211>  355
<212>  PRT
<213>  Aequorea victoria

<400>  2

Met Gly Ala Arg Ala Ser Val Leu Ser Gly Gly Glu Leu Asp Arg Trp
1               5                   10                  15

Glu Lys Ile Arg Leu Arg Pro Gly Gly Lys Lys Lys Tyr Lys Leu Lys
            20                  25                  30

His Ile Val Trp Ala Ser Arg Glu Leu Glu Arg Phe Ala Val Asn Pro
        35                  40                  45

Gly Leu Leu Glu Thr Ser Glu Gly Cys Arg Gln Ile Leu Gly Gln Leu
    50                  55                  60

Gln Pro Ser Leu Gln Thr Gly Ser Glu Glu Leu Arg Ser Leu Tyr Asn
65                  70                  75                  80

Thr Val Ala Thr Leu Tyr Cys Val His Gln Arg Ile Glu Ile Lys Asp
                85                  90                  95

Thr Lys Glu Ala Ser Asn Ser Ala Val Asp Gly Thr Ala Gly Pro Gly
            100                 105                 110

Ser Ile Ala Thr Met Val Ser Lys Gly Glu Glu Leu Phe Thr Gly Val
        115                 120                 125

Val Pro Ile Leu Val Glu Leu Asp Gly Asp Val Asn Gly His Lys Phe
    130                 135                 140

Ser Val Ser Gly Glu Gly Glu Gly Asp Ala Thr Tyr Gly Lys Leu Thr
145                 150                 155                 160

Leu Lys Phe Ile Cys Thr Thr Gly Lys Leu Pro Val Pro Trp Pro Thr
                165                 170                 175

Leu Val Thr Thr Leu Thr Tyr Gly Val Gln Cys Phe Ser Arg Tyr Pro
            180                 185                 190

Asp His Met Lys Gln His Asp Phe Phe Lys Ser Ala Met Pro Glu Gly
        195                 200                 205

Tyr Val Gln Glu Arg Thr Ile Phe Phe Lys Asp Asp Gly Asn Tyr Lys
    210                 215                 220

Thr Arg Ala Glu Val Lys Phe Glu Gly Asp Thr Leu Val Asn Arg Ile
225                 230                 235                 240

Glu Leu Lys Gly Ile Asp Phe Lys Glu Asp Gly Asn Ile Leu Gly His
                245                 250                 255

Lys Leu Glu Tyr Asn Tyr Asn Ser His Asn Val Tyr Ile Met Ala Asp
            260                 265                 270

Lys Gln Lys Asn Gly Ile Lys Val Asn Phe Lys Ile Arg His Asn Ile
        275                 280                 285

Glu Asp Gly Ser Val Gln Leu Ala Asp His Tyr Gln Gln Asn Thr Pro
    290                 295                 300

Ile Gly Asp Gly Pro Val Leu Leu Pro Asp Asn His Tyr Leu Ser Thr
305                 310                 315                 320

Gln Ser Ala Leu Ser Lys Asp Pro Asn Glu Lys Arg Asp His Met Val
                325                 330                 335

Leu Leu Glu Phe Val Thr Ala Ala Gly Ile Thr Leu Gly Met Asp Glu
            340                 345                 350

Leu Tyr Lys
        355

<210>  3
<211>  9681
<212>  DNA
<213>  Artificial Sequence

<220>
<223>  Plasmid sequence

<220>
<221>  CDS
<222>  (5009)..(5692)

<400>  1
ttctcatgtt tgacagctta tcatcgcaga tccgggcaac gttgttgcca ttgctgcagg      60

cgcagaactg gtaggtatgg aagatctata cattgaatca atattggcaa ttagccatat     120

tagtcattgg ttatatagca taaatcaata ttggctattg gccattgcat acgttgtatc     180

tatatcataa tatgtacatt tatattggct catgtccaat atgaccgcca tgttgacatt     240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtccgccc cctattgacg tcaatgacgg taaatggccc gcctggcatt 540

atgcccagta catgacctta cgggactttc ctacttggca gtacatctac gtattagtca 600

tcgctattac catggtgatg cggttttggc agtacaccaa tgggcgtgga tagcggtttg 660

actcacgggg atttccaagt ctccacccca ttgacgtcaa tgggagtttg ttttggcacc 720

aaaatcaacg ggactttcca aaatgtcgta ataaccccgc cccgttgacg caaatgggcg 780

gtaggcgtgt acggtgggag gtctatataa gcagagctcg tttagtgaac cgtcagatct 840

ctagaagctg gctgggccgg cttggcctcc attgagatcc ggaggtgagc tggcggacgc 900

tggggaccgg cgggacagcg ggtctcgcgc tccgcctggg ctcacgaggc ggctgcgaac 960

cgaggggacc gcgaggacac gtccttgagg cccggcggca gggcggcggc cagggaaccc 1020

gtgggccggg tgggcctgag ggccagtgca gcgacggggc gtccgtgggt tcagtccgga 1080

gggcgctgtg ctgacccggc ggtcacggtc atccgtgtac gagtgcctgg cagaggggac 1140

agcgggagcc ctagagaaca agggttcacg cccaggcgct gccttgcggc aggacccgtc 1200

tttaaagtga tggagatgta ggctgcaaat tgacccgatc cgcgtgggta cggcaggttg 1260

tgctcttcct agtgggcccg gaggaggatg cgaggtctgt acaggcacag ctagccctcc 1320

gaagtcggtc tctcgacctt tatgttatag tctttctcct aaggggcgta gtctcattga 1380

tactgatgtc aatttacctt atgcacctat tggaattatt tggtttgaat gaagaaaatg 1440

gccttagagg ttcgcaagta aaacagtaga cttctgaggt tgggcgaagt taatagtctt 1500

tcatcgaaag cggacagatg gaaatctttc ctctcttgag gttgtatctt ttcgggctaa 1560

ggcctgtaaa ggcacaggaa acgtctgtta ctggctcttt acttacaaag tcgttgtgta 1620

caattaaagc atcacacgaa gtagggaata cattttgttt ggttacttgt gatttatttg 1680

tattttattt atcgtagggc aaaagtcttg gatctacatt gggctgcgtt ttgtgggctt 1740

acttggtcgt agtgcctttc tagaacttga ccaggcaaga taaagggtgt gcttgctcac 1800

actgctatat ttttgtctaa tttgtagact gtgagctata ttctgggatc agtttctgaa 1860

tccttaaatc tatcttgaaa ttgtgctttt tttttttttt ttaagttctt aaaatagtag 1920

ttctttatgg ttccacttaa tactttggtc agagaacttg accatgaaga ggagatggac 1980

ttagaaactg gtaatgtatg gtaaagttgg gtacagaact tgttttctgt gcaaggtgat 2040

gatggccccc tttgatggat gggtaggtta agaatgctga gtgttcatcc tgttgggatt 2100

agacgtggct acagtgtagg attagggggt cttaccctca aagggacaac ctgcacatat 2160

cagtgtagat attttctaat caaagagtgg gttgtgtact accggaagaa tgaatggact 2220

ggaagaatct cagagaagcc tgtctcttgg cagtggttgg tggttctatt cctagataga 2280

attagaattt aagtctggtg agtttaaatt ttcagccatt tgaggagtct gtgaaatgtt 2340

tgctgtatac tgtatttgga taagtaggca agttctttta ttgcagcgca gcagaaaagg 2400

gaccttccga gctttcataa actgctacac cggtgtcact ttgcctagtc acagagaaat 2460

gggaggtgct cctgagagac aagtttataa ttacggttac ttttgacata cctttgtaac 2520

actgctcagt ttgtagttcc aggtaggtgt ggttctctgt gcttggttgg attgtggaac 2580

aggattttgt gtaatttttt cccctcagat tatttaataa taataaaaaa agacaatgta 2640

cgtgcaggga agagtggcaa gattgccagg ccccgggaac cgagcagggt gggaggtctg 2700

gtgtgcctgg ggtagtgtgg gtatggggtt actagttggc catcacattg gggctcctca 2760

tgatagcaat ttacattttt ctcagcggaa tcacttttaa tagtgatgaa taaaattgac 2820

tcgttaagga agactattga agctttgtgg tgaagggtgg aggactggaa gtgctttaga 2880

tttacatttg aaactaattt accttctgaa accttgaagc cagtcttttc caacagtttg 2940

ctttagtttc tgattttaag agattctgtg agagaggcca gtggacatgt ggtttctcat 3000

gagaatccta gacaggttta aagtgtttgt tctttaaacc caggccctcc ataaaagtac 3060

tgtgtggttg tcctttcttg ggtatttgtg tacattagtg caagatgccg gagaggaaag 3120

tggtaaaact cattatgcag tactgaaaaa aagataaata ctgatgcctt ccacatctta 3180

tgccatacta gttgttcttt ccaaaattaa gacccacagg aagaaatata gtgagaatgt 3240

tgtgaatgat ttcatgacgc atgagtttac atggtactta cccgtgttcc ctgccttacc 3300

tgtcaactac ttttatgttg ctttctattt tttagttaat taaaactgtt atcagcaaca 3360

ttgattttta tgattgaatt aatgtgttgc tctgactatc ttgaaaacca gttatattgg 3420

cgtgtagcct ggtatattga aatatagagg aaaaatggaa tgttggaatc agagatgatg 3480

ttggctaatt ggcttcattc ttaaaagcca agcccccacc cttcttaccc cccaccctcg 3540

ccctagctac cacccttctt acctcccacc cttgccccag ctcccaccgt tcttaccccc 3600

accctcgccc cagtactttt ttgcctcctt taatttggtg gtggtggtcc cttttttttt 3660

gttttgtttt gtttttttcc agacagggtt tctctgtgta gcccagactg gctttgagct 3720

cagagatctg actgcctctg cctcccttgt caccactgcc tggctagttg gtgtcatcag 3780

tcaataagtc actttcctag tagatgtgtg agatctattt gaattcttgc tgacaatcag 3840

tgggctgtta gttggtttct tgtcatcatt tgtattctat tgtgtgtgtt tgtgtgcact 3900

ggtgtgtact gtggcctgag cgtggcggtc agagctcagt ctgcagtgtt agaagcagtg 3960

aggcttagat gcaagtgcat tatccacagg cccatctccc ggggtctagt ttttgtgact 4020

gattgttatg ggtaatagtt ttcattttgc tctatagttt taccatgaga aatatctctg 4080

taggccatca gactataaga ttacatttca aataggaatg tcaaacttgt taattagcca 4140

tcagatgtgg gaagggtggc agtcatttca ttagctcaga tcttgctatt tattctgttg 4200

aatcctcttg tcagacaaga agtcgtagtt ttgggggttt tttgaacaga gggccctgca 4260

atgagctgta tcttcactcc ttgggggtct ttctgtatga tacatacatg tttcatttta 4320

cccagcatct acttctactg actttccagt cttggctggc tgtaaaagtt taggagcctt 4380

ctacatctaa cacatttaaa tgggccttct tgcccctgta ctttgccagg agaaaagcca 4440

tcctagtgaa atgcatgccc ccgttttcct aatgtggaag tatagatcag aaaagatttc 4500

tggtgacagc caccttaggg aagttcctta tacattgtgc tagtgacatt ctgccttatt 4560

ctagagactg aaatgaaaag caggatttat ggcaaggtgc tcgagaagaa ggttctttgg 4620

tgttgccttt attaggcatg tggtggtgaa gaggatgctc gtgggctttc tgtttaacta 4680

aaaagtggga atagtctcat gtctgctttg tgctttattt attctgctcc tccttggctc 4740

acataactac aaggagttac tcttgaaact taacttaaag gaacatttgc atataagttg 4800

tttaccagtg agtaataagg gatttaggtg gttgaagaag tggcaagcac cgtgttaata 4860

gagcattaca ctcactttcc aggattatgc agaacgctgg aatagtcctg tacatgagat 4920

gctgcactta gggctgaaag tgagccccat gcatgctaag tgttcttcat atgtatatcc 4980

ttccaggatc tggatccagc ttttcaaa atg gaa gac gcc aaa aac ata aag 5032
Met Glu Asp Ala Lys Asn Ile Lys
1               5

aaa ggc ccg gcg cca ttc tat cct cta gag gat gga acc gct gga gag 5080
Lys Gly Pro Ala Pro Phe Tyr Pro Leu Glu Asp Gly Thr Ala Gly Glu
    10                  15                  20

caa ctg cat aag gct atg aag aga tac gcc ctg gtt cct gga aca att 5128
Gln Leu His Lys Ala Met Lys Arg Tyr Ala Leu Val Pro Gly Thr Ile
25                  30                  35                  40

gct ttt aca gat gca cat atc gag gtg aac atc atg tac gcg gaa tac 5176
Ala Phe Thr Asp Ala His Ile Glu Val Asn Ile Met Tyr Ala Glu Tyr
                45                  50                  55

ttc gaa atg tcc gtt cgg ttg gca gaa gct atg aaa cga tat ggg ctg 5224
Phe Glu Met Ser Val Arg Leu Ala Glu Ala Met Lys Arg Tyr Gly Leu
            60                  65                  70

aat aca aat cac aga atc gtc gta tgc agt gaa aac tct ctt caa ttc 5272
Asn Thr Asn His Arg Ile Val Val Cys Ser Glu Asn Ser Leu Gln Phe
        75                  80                  85

ttt atg ccg gtg ttg ggc gcg tta ttt atc gga gtt gca gtt gcg ccc 5320
Phe Met Pro Val Leu Gly Ala Leu Phe Ile Gly Val Ala Val Ala Pro
    90                  95                  100

gcg aac gac att tat aat gaa cgt gaa ttg ctc aac agt atg aac att 5368
Ala Asn Asp Ile Tyr Asn Glu Arg Glu Leu Leu Asn Ser Met Asn Ile

105                 110                 115                 120

tcg cag cct acc gta gtg ttt gtt tcc aaa aag ggg ttg caa aaa att       5416
Ser Gln Pro Thr Val Val Phe Val Ser Lys Lys Gly Leu Gln Lys Ile
                125                 130                 135

ttg aac gtg caa aaa aaa tta cca ata atc cag aaa att att atc atg       5464
Leu Asn Val Gln Lys Lys Leu Pro Ile Ile Gln Lys Ile Ile Ile Met
            140                 145                 150

gat tct aaa acg gat tac cag gga ttt cag tcg atg tac acg ttc gtc       5512
Asp Ser Lys Thr Asp Tyr Gln Gly Phe Gln Ser Met Tyr Thr Phe Val
        155                 160                 165

aca tct cat cta cct ccc ggt ttt aat gaa tac gat ttt gta cca gag       5560
Thr Ser His Leu Pro Pro Gly Phe Asn Glu Tyr Asp Phe Val Pro Glu
    170                 175                 180

tcc ttt gat cgt gac aaa aca att gca ctg ata atg aat tcc tct gga       5608
Ser Phe Asp Arg Asp Lys Thr Ile Ala Leu Ile Met Asn Ser Ser Gly
185                 190                 195                 200

tct act ggg tta cct aag ggt gtg gcc ctt ccg cat aga act gcc tgc       5656
Ser Thr Gly Leu Pro Lys Gly Val Ala Leu Pro His Arg Thr Ala Cys
                205                 210                 215

gtc aga ttc tcg cat gcc aga gat cct att ttt ggc aatcaaatca            5702
Val Arg Phe Ser His Ala Arg Asp Pro Ile Phe Gly
            220                 225

ttccggatac tgcgatttta agtgttgttc cattccatca cggttttgga atgtttacta     5762

cactcggata tttgatatgt ggatttcgag tcgtcttaat gtatagattt gaagaagagc     5822

tgtttttacg atcccttcag gattacaaaa ttcaaagtgc gttgctagta ccaaccctat     5882

tttcattctt cgccaaaagc actctgattg acaaatacga tttatctaat ttacacgaaa     5942

ttgcttctgg gggcgcacct ctttcgaaag aagtcgggga agcggttgca aaacgcttcc     6002

atcttccagg gatacgacaa ggatatgggc tcactgagac tacatcagct attctgatta     6062

cacccgaggg ggatgataaa ccgggcgcgg tcggtaaagt tgttccattt tttgaagcga     6122

aggttgtgga tctggatacc gggaaaacgc tgggcgttaa tcagagaggc gaattatgtg     6182

tcagaggacc tatgattatg tccggttatg taaacaatcc ggaagcgacc aacgccttga     6242

ttgacaagga tggatggcta cattctggag acatagctta ctgggacgaa gacgaacact     6302

tcttcatagt tgaccgcttg aagtctttaa ttaaatacaa aggatatcag gtggcccccg     6362

ctgaattgga atcgatattg ttacaacacc ccaacatctt cgacgcgggc gtggcaggtc     6422

ttcccgacga tgacgccggt gaacttcccg ccgccgttgt tgttttggag cacggaaaga     6482

cgatgacgga aaaagagatc gtggattacg tcgccagtca agtaacaacc gcgaaaaagt     6542

tgcgcggagg agttgtgttt gtggacgaag taccgaaagg tcttaccgga aaactcgacg     6602

caagaaaaat cagagagatc ctcataaagg ccaagaaggg cggaaagtcc aaattgtaaa     6662

agaattcttt ctgctaggat ccagacatga taagatacat tgatgagttt ggacaaacca     6722

caactagaat gcagtgaaaa aaatgcttta tttgtgaaat ttgtgatgct attgctttat 6782

ttgtaaccat tataagctgc aataaacaag ttaacaacaa caattgcatt cattttatgt 6842

ttcaggttca gggggaggtg ggaggttttt ttaaagcaag taaaacctct acaaatgtgg 6902

tatggctgat tatgatccgg ctgcctcgcg cgtttcggtg atgacggtga aaacctctga 6962

cacatgcagc tcccggagac ggtcacagct tgtctgtaag cggatgccgg gagcagacaa 7022

gcccgtcagg gcgcgtcagc gggtgttggc gggtgtcggg gcgcagccat gacgtcgact 7082

ctaccggaaa tacaggaacg cacgctggat ggcccttcgc tgggatggtg aaaccatgaa 7142

aaatggcagc ttcagtggat taagtggggg taatgtggcc tgtaccctct ggttgcatag 7202

gtattcatac ggttaaaatt tatcaggcgc gatcgcggca gtttttcggg tggtttgttg 7262

ccatttttac ctgtctgctg ccgtgatcgc gctgaacgcg ttttagcggt gcgtacaatt 7322

aagggattat ggtaaatcca cttactgtct gccctcgtag ccatcgtaga gcggccctag 7382

ctacgtagat gataagctgt caaacatgag aattcttgaa gacgaaaggg cctcgtgata 7442

cgcctatttt tataggttaa tgtcatgata ataatggttt cttagacgtc aggtggcact 7502

tttcggggaa atgtgcgcgg aacccctatt tgtttatttt tctaaataca ttcaaatatg 7562

tatccgctca tgagacaata accctgataa atgcttcaat aatattgaaa aaggaagagt 7622

atgagccata ttcaacggga aacgtcttgc tcgaggccgc gattaaattc caacatggat 7682

gctgatttat atgggtataa atgggctcgc gataatgtcg ggcaatcagg tgcgacaatc 7742

tatcgattgt atgggaagcc cgatgcgcca gagttgtttc tgaaacatgg caaaggtagc 7802

gttgccaatg atgttacaga tgagatggtc agactaaact ggctgacgga atttatgcct 7862

cttccgacca tcaagcattt tatccgtact cctgatgatg catggttact caccactgcg 7922

atccccggga aaacagcatt ccaggtatta gaagaatatc ctgattcagg tgaaaatatt 7982

gttgatgcgc tggcagtgtt cctgcgccgg ttgcattcga ttcctgtttg taattgtcct 8042

tttaacagcg atcgcgtatt tcgtctcgct caggcgcaat cacgaatgaa taacggtttg 8102

gttgatgcga gtgattttga tgacgagcgt aatggctggc ctgttgaaca agtctggaaa 8162

gaaatgcata agcttttgcc attctcaccg gattcagtcg tcactcatgg tgatttctca 8222

cttgataacc ttatttttga cgaggggaaa ttaataggtt gtattgatgt tggacgagtc 8282

ggaatcgcag accgatacca ggatcttgcc atcctatgga actgcctcgg tgagttttct 8342

ccttcattac agaaacggct ttttcaaaaa tatggtattg ataatcctga tatgaataaa 8402

ttgcagtttc atttgatgct cgatgagttt ttctaatcag aattggttaa ttggttgtaa 8462

cactggcaga gcattacgct gacttgacgg gacggcggct ttgttgaata aatcgaactt 8522

ttgctgagtt gaaggatcag atcacgcatc ttcccgacaa cgcagaccgt tccgtggcaa 8582

agcaaaagtt caaaatcacc aactggtcca cctacaacaa agctctcatc aaccgtggct 8642

ccctcacttt ctggctggat gatggggcga ttcaggcctg gtatgagtca gcaacacctt 8702

cttcacgagg cagagatctc ccttaacgtg agttttcgtt ccactgagcg tcagaccccg 8762

tagaaaagat caaaggatct tcttgagatc ctttttttct gcgcgtaatc tgctgcttgc 8822

aaacaaaaaa accaccgcta ccagcggtgg tttgtttgcc ggatcaagag ctaccaactc 8882

tttttccgaa ggtaactggc ttcagcagag cgcagatacc aaatactgtc cttctagtgt 8942

agccgtagtt aggccaccac ttcaagaact ctgtagcacc gcctacatac ctcgctctgc 9002

taatcctgtt accagtggct gctgccagtg gcgataagtc gtgtcttacc gggttggact 9062

caagacgata gttaccggat aaggcgcagc ggtcgggctg aacggggggt tcgtgcacac 9122

agcccagctt ggagcgaacg acctacaccg aactgagata cctacagcgt gagctatgag 9182

aaagcgccac gcttcccgaa gggagaaagg cggacaggta tccggtaagc ggcagggtcg 9242

gaacaggaga gcgcacgagg gagcttccag ggggaaacgc ctggtatctt tatagtcctg 9302

tcgggtttcg ccacctctga cttgagcgtc gatttttgtg atgctcgtca ggggggcgga 9362

gcctatggaa aaacgccagc aacgcggcct ttttacggtt cctggccttt tgctggcctt 9422

gaagctgtcc ctgatggtcg tcatctacct gcctggacag catggcctgc aacgcgggca 9482

tcccgatgcc gccggaagcg agaagaatca taatggggaa ggccatccag cctcgcgtcg 9542

cgaacgccag caagacgtag cccagcgcgt cggccccgag atgcgccgcg tgcggctgct 9602

ggagatggcg gacgcgatgg atatgttctg ccaagggttg gtttgcgcaa gaagagcggc 9662

ctgtgtggcc gtcgaccaa 9681

<210> 4
<211> 228
<212> PRT
<213> Artificial Sequence

<220>
<223> Synthetic Construct

<400> 2

Met Glu Asp Ala Lys Asn Ile Lys Lys Gly Pro Ala Pro Phe Tyr Pro
1               5                   10                  15

Leu Glu Asp Gly Thr Ala Gly Glu Gln Leu His Lys Ala Met Lys Arg
            20                  25                  30

Tyr Ala Leu Val Pro Gly Thr Ile Ala Phe Thr Asp Ala His Ile Glu
        35                  40                  45

Val Asn Ile Met Tyr Ala Glu Tyr Phe Glu Met Ser Val Arg Leu Ala
    50                  55                  60

Glu Ala Met Lys Arg Tyr Gly Leu Asn Thr Asn His Arg Ile Val Val
65                  70                  75                  80

Cys Ser Glu Asn Ser Leu Gln Phe Phe Met Pro Val Leu Gly Ala Leu
                85                  90                  95

Phe Ile Gly Val Ala Val Ala Pro Ala Asn Asp Ile Tyr Asn Glu Arg
            100                 105                 110

Glu Leu Leu Asn Ser Met Asn Ile Ser Gln Pro Thr Val Val Phe Val
        115                 120                 125

Ser Lys Lys Gly Leu Gln Lys Ile Leu Asn Val Gln Lys Lys Leu Pro
    130                 135                 140

Ile Ile Gln Lys Ile Ile Ile Met Asp Ser Lys Thr Asp Tyr Gln Gly
145                 150                 155                 160

Phe Gln Ser Met Tyr Thr Phe Val Thr Ser His Leu Pro Pro Gly Phe
                165                 170                 175

Asn Glu Tyr Asp Phe Val Pro Glu Ser Phe Asp Arg Asp Lys Thr Ile
            180                 185                 190

Ala Leu Ile Met Asn Ser Ser Gly Ser Thr Gly Leu Pro Lys Gly Val
        195                 200                 205

Ala Leu Pro His Arg Thr Ala Cys Val Arg Phe Ser His Ala Arg Asp
    210                 215                 220

Pro Ile Phe Gly
225

## Description

The present invention provides a new transfection agent for use as a carrier for nucleic acid transfer, a polyp lex comprising the transfection agent and nucleic acid, a pharmaceutical composition for use in gene therapy and a method for transferring nucleic acid into a cell.

Gene transfer consists in the introduction of genetic materials (DNA or RNA) inside a cell nucleus in order to restore or modify the cellular functions. When used in the frame of therapeutic treatments, such gene transfer methods are referred to as "gene therapy". A wide range of methods have been used to protect the genetic material during the transfection for maximizing the amount of plasmid internalized into the cells and eventually within the nuclei.

Transfection agents can roughly be divided in two main categories, viral and non-viral carriers. The viral gene transfer approach usually offers the highest transfection efficacy but suffers from severe limitations due to the toxicity of the viral-based materials and their tendency to trigger immune diseases and disorders. Production of virus in large scale is also an expensive task that does not allow today envisioning the treatment of disease for a large cohort of patients. By contrast, non-viral agents exhibit more modest transfection performances but generate no or limited immune responses. These include cationic polymers which form polymer-polynucleotide nanoparticles complexes, called polyplexes. The complexation of the DNA by a positively charged synthetic macromolecule facilitates the interaction of the resulting polyplex with the cell membrane and so its internalization. Most polyplexes are internalized through a clathrin-dependant endosome pathway. The subsequent steps of transfection are the escape of the polyplex from the endosome into the cytosol, notably by a proton sponge mechanism, the decomplexation of the polynucleotide and ultimately its internalization within the nuclei. This last step is not required for RNA transfection.

In the last decades, a wide range of polycations with different repeating units, macromolecular parameters, architectures and functions has been tested. The most efficient include linear and branched polyethylene imine derivatives (1PEI and bPEI, respectively). The 1PEI is currently considered as a reference due to its largely satisfactory transfection capacity. Nevertheless, the inherent toxicity of this polycation remains a crucial concern and issue. In this respect, the substitution of the 1PEI by hydrophobic moieties or by histidine has been found to lead to a reduction of the toxicity of the vector without drastically affecting their transfection performance. On the other hand, the search for novel cationic polymers with low toxicity and high transfection ability is still ongoing. For example, polyvinylamine (PVAm), an isomer of the linear polyethylene imine, has also been considered for the gene transfection application. PVAms with poorly defined molecular parameters were obtained by free radical polymerization (FRP) of N-vinylformamide followed by deprotection of the amide moieties under hydrolytic conditions. Nevertheless, the transfection efficiency of the PVAm-based agents proved to be disappointing and, from that time on, this class of polymer has been disregarded.

A way of ameliorating these problems has been sought.

According to the invention there is provided a polyplex comprising:
(a) at least one polymer of formula (I) comprising at least one set of repeating units of formula: 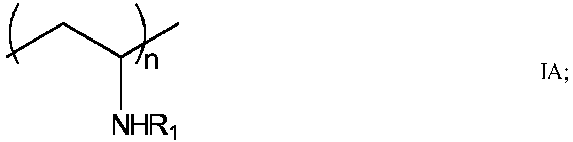 and 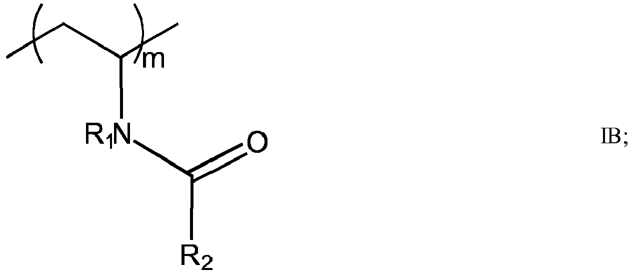
   wherein for each set of repeating units R₁ represents either a hydrogen atom or a straight or branched chain alkyl group having from 1 to 20 carbon atoms; and R₂ represents a straight or branched chain alkyl group having from 1 to 20 carbon atoms,
   wherein n and m each represent an integer of from 1 to 5000,
   wherein the sum of n and m is from 10 to 250 when R₁ represents a hydrogen atom and when the polymer of formula (I) contains only one set of repeating units; and
(b) at least one nucleic acid. In some embodiments, R₁ may represent methyl or ethyl.

According to the invention there is further provided a method for preparing a polyplex which method comprises the step of combining at least one nucleic acid with at least one polymer of formula (I) as defined above.

According to the invention there is also provided a method for transferring at least one nucleic acid into a cell which method comprises the step of contacting the cell with at least one polyplex according to the invention.

According to the invention there is further provided a pharmaceutical composition comprising a polyplex according to the invention and an adjuvant.

According to the invention there is also provided a method for preparing a polymer of formula (I) for use in a polyplex according to the invention wherein the method comprises one or more of the following steps:
(i) polymerization of a monomer of formula (IC) 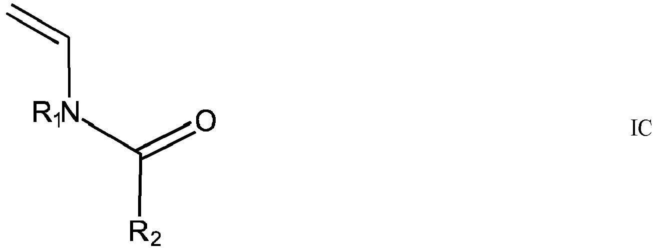 wherein R₁ and R₂ are as defined for the polymer of formula (I) followed by hydrolysis, and/or
(ii) polymerization of a monomer of formula (IC) as defined in (i) followed by reduction of the amide function. In some embodiments, the monomer of formula (IC) may be N-vinylacetamide, N-methyl-N-vinylacetamide, N-ethyl-N-vinylacetamide.

According to the invention there is further provided use of a polyplex according to the invention or of a pharmaceutical composition according to the invention for transferring at least one nucleic acid into a cell.

According to the invention there is also provided use of a polymer of formula (I) as defined above in the transfer of at least one nucleic acid into a cell.

Herein, a polymer of formula (I) which comprises one set of repeating units wherein R₁ represents a hydrogen atom is referred to as being a primary polyvinylamine comprising a repeating unit having an amide group (PVAAm); a polymer of formula (I) which comprises one set of repeating units wherein R₁ represents an alkyl group is referred to as being a secondary poly(N-alkylvinylamine) comprising a repeating unit having an amide group (PAVAAm).

Advantages of the invention include that it has surprisingly been found that the polyplex according to the invention has a high transfection efficiency and a low cytotoxicity.

Surprisingly, compared to previous studies reported by Wolfert *et al.* [2], high transfection levels (close to 1PEI) were obtained with the polyplexes comprising the defined polyvinylamines comprising an amide group. A high efficiency with low molar mass PVAAm-based polymers was observed. In the study of Wolfert *et al*., a PVAm polymer was tested which is not a polymer according to formula (I) because of the lack of N-vinylacetamide monomers. The Wolfert *et al*. PVAm was obtained by alkaline hydrolysis of polyvinylformamide. They established that their PVAm was a poor transfecting agent, reaching lower transfection efficiency (< 10⁵ RLU/mg proteins) compared to poly(L-lysine) tested in the same study (10⁸ RLU/mg proteins, using pDNA coding for β-galactosidase activity) and which is known to be less efficient than 1PEI. Interestingly, Wolfert *et al*. also tested their PVAm with three different molar masses (3, 8 and 60 kg/mol, corresponding to a degree of polymerization (DPn) of 38, 101 and 760). Their results were strictly opposite to the findings of the present invention: higher molar mass reached the best Transfection efficiency and had a higher cytotoxicity whereas the shortest PVAm used in the invention prepared by organometallic-mediated radical polymerisation (OMRP) (DPn 139) was found to give the highest transfection and best cell viability.

In the present invention, a series of polyvinylamines comprising an amide group (PVAAm) and poly(N-alkylvinylamines) comprising an amide group (PAVAAm) called homopolymers possessing pendant primary and secondary amines respectively, were synthesized for use in the polyplex according to the invention. These two types of polymers can be potential nucleic acid carriers thanks to their backbone having a high amine density.

In some embodiments, the polyplex according to the invention comprises a polymer of formula (I) which comprises one set of repeating units wherein R₁ represents a straight or branched chain alkyl group having from 1 to 20 carbon atoms. In some embodiments, R₁ represents a straight or branched chain alkyl group having from 1 to 20, for example to 16, 12, 8 or to 4 carbon atoms, especially from 1 to 5 carbon atoms. In some embodiments, R₁ represents a methyl group. As can be seen from comparing the data for compound 1 and compound 2 in Examples 1 and 2, which have quite similar degree of polymerization as defined by the sum of the integers n and m (DPn ∼150) and similar composition in repeating units, the substitution of one hydrogen by a hydrophobic methyl group, yielding secondary polyvinylamines, permitted reproducibly improving the transfection efficiency by a factor of 10 (respectively 10⁸ and 10⁹ RLU/mg proteins).

In some embodiments, the polymer of formula (I) may be a copolymer of formula (I) comprising repeating units (IA), (IB) and one or more additional repeating units. It was found that a copolymer of formula (I) was also effective in forming polyplexes according to the invention. Herein a copolymer of formula (I) comprising repeating units (IA), (IB), (IA') and (IB') wherein R₁ represents a hydrogen atom for the set of repeating units (IA) and (IB) and wherein R₁ represents a straight or branched chain alkyl group comprising from 1 to 20 carbon atoms for the set of repeating units (IA') and (IB') is referred to as copolymers, named P(VAAm-*co*-AVAAm). Results of transfection with polyplexes of P(VAAm-*co*-AVAAm) were positive, with the same range of transfection efficiency as 1PEI and lower cytotoxicity. Compared to the homopolymers (PVAAm and PAVAAm), P(VAAm-*co*-AVAAm) was at least as efficient and even more efficient on muscular and dendritic cells.

In some embodiments, the copolymer of formula (I) may be a block copolymer or a statistical copolymer. The copolymer of formula (I) may also be of any other structures such as random, grafted or tapered structure.

In some embodiments, the polymer of formula (I) may be a copolymer or terpolymer including a repeating unit of formula : 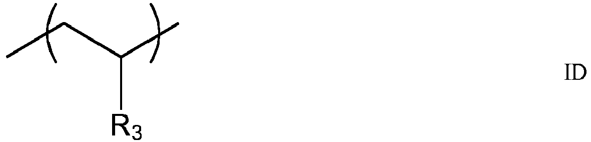 wherein R₃ represents an optionally substituted alkyl, aryl, heterocycle, alcohol, ester or amino-acid containing group. In some embodiments, an alkyl group represented by R₃ may be a straight or branched chain, saturated or unsaturated alkyl group comprising from 1 to 12, for example to 8 carbon atoms. In some embodiments, an aryl group represented by R₃ may be any monocyclic or polycyclic substituent derived from an aromatic ring, for example comprising from 5 to 12 carbon atoms. In some embodiments, an a heterocycle group represented by R₃ may be any monocyclic or polycyclic, saturated or unsaturated group comprising from 3 to 12 group members comprising a carbon atom and at least one hetero atom (particularly nitrogen), for example pyrrole, imidazole, pyridine or pyrazole. In some embodiments, R₃ represents a derivative of an amino acid, for example arginine or histidine.

It was found that a copolymer of formula (I) comprising a vinylimidazole repeating group (VIm) of formula (ID) formed an effective polyplex. Such a copolymer of formula (I) comprising a VIm group may be a P(VAAm-co-VIm) copolymer, a P(AVAAm-*co*-VIm) copolymer or a P(VAAm-*co*-AVAAm-*co*-VIm) copolymer. The copolymers P(AVAAm-*co*-VIm) were found to be efficient as nucleic acid carriers, with high transfection efficiency and low cytotoxicity. Such copolymers may be synthesised by copolymerization of NAVA and/or NVA (*N-*vinylacetamide) with 4-vinylimidazole (4VIm) followed by acidic hydrolysis.

In some embodiments, the polymer of formula (I) may be a copolymer or terpolymer including a repeating unit of formula: 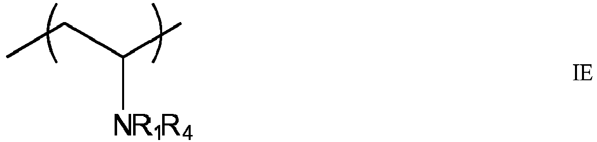 wherein R₁ is as defined above and R₄ represents a hydrogen atom, an optionally substituted alkyl, aryl or heterocycle group or R₁ and R₄ together form a heterocycle group. In some embodiments, an alkyl group represented by R₄ may be a straight or branched chain, saturated or unsaturated alkyl group comprising from 1 to 12, for example to 8 carbon atoms. In some embodiments, an aryl group represented by R₄ may be any monocyclic or polycyclic substituent derived from an aromatic ring, for example comprising from 5 to 12 carbon atoms. In some embodiments, a heterocycle group represented by R₄ or by R₁ and R₄ together may be any monocyclic or polycyclic, saturated or unsaturated group comprising from 3 to 12 group members comprising a carbon atom and at least one hetero atom (particularly nitrogen), for example pyrrole, imidazole, pyridine or pyrazole. In some embodiments, R₄ represents a derivative of an amino acid, for example arginine or histidine.

In some embodiments, the method of preparing the polymer of formula (I) may comprise step (i) and optionally step (ii). In some embodiments, a PAVAAm polymer may be synthesised via either (i) polymerization of NAVA (*N*-alkylvinylacetamide) by a free or controlled radical process followed by acidic or basic hydrolysis, and/or (ii) polymerization of NAVA by a free or controlled radical process followed by reduction of the amide function. For example, where the alkyl group is ethyl, a PNEtVA (poly(*N*-ethylinylamine) copolymer may be synthesised via either (i) polymerization of NEtVA (*N*-ethylvinylacetamide) by a free or controlled radical process followed by acidic or basic hydrolysis, or (ii) polymerization of NVA by a free or controlled radical process followed by partial reduction of the amide function and optionally, hydrolysis of part of the residual amides.

In some embodiments, the polymer of formula (I) used in the polyplex according to the invention is obtained by the method of preparing the polymer of formula (I) according to the invention.

It was surprisingly observed that the presence of a few amide functions along the polymer chain improved transfection. This factor is not disclosed in the prior art. To confirm this effect, a comparative example was done with a polyvinylamine synthesized by a technique of the prior art. That technique yielded a polyvinylamine comprising repeating unit of formula IB . The transfection efficiency was significantly lower than that obtained with a comparable polymer of formula (I).

In some embodiments, the polymer of formula (I) was obtained by performing a substantially partial conversion of the amide functions into amine functions. The conversion of the amide functions may be done by amidolysis or hydrolysis. The hydrolysis level is a percentage calculated by multiplying the ratio of n:(n+m) by 100. In some embodiments, n represents an integer which is greater than that represented by m such that the hydrolysis level is from 50%, for example from 70%, 75%, 80% or from 85% to 97%, for example to 90%. To understand the effect of the hydrolysis level, PNAVA was also hydrolyzed with soft conditions (2N, 120°C, 14h) in order to obtain low hydrolyzed PAVAAm. Diminishing the number of amine functions reduces the number of positive charges along the chain. It was found that slightly higher pDNA/polymer weight ratios (of from 1/2 to 1/8) were needed to complex the pDNA by the relatively low hydrolyzed PAVAAm compared to the relatively highly hydrolyzed PAVAAm (1/1 to 1/6). The presence of a large percentage of amide units might conduct to a weaker cohesion between the pDNA and the polymer of formula (I), and therefore to a poor protection of the genetic material and to an early release. The tests on homopolymers showed that hydrolysis level from 70% to 95% gave good results whereas a hydrolysis level of 19% and 25% gave poor results.

In some embodiments, the copolymer of formula (I) which is P(VAAm-co-AVAAm) has a hydrolysis level for the different sets of repeating units which may be the same or different. For example, a hydrolysis level giving good performances was above 20 % for the set of repeating units (IA') and (IB') (secondary amines), in a copolymer P(VAAm-co-AVAAm) where the molar ratio was 50/50 for the set of repeating units (IA) and (IB) to the set of repeating units (IA') and (IB') (see compound 10). For example, levels of hydrolysis from 90% for the set of repeating units (IA) and (IB) (NVA) and 20-75% for the set of repeating units (IA') and (IB') (NAVA). In some embodiments, a copolymer of formula (I) which is P(VAAm-co-AVAAm) may have a total hydrolysis level of from 50% to 80%. It was found that this hydrolysis level gave better results compared to a higher hydrolysis level. Indeed it was found that a hydrolysis level of up to 94% for NVA and from 75% to 90% for NAVA such that the total hydrolysis level was from 80 to 90%, resulted in lower transfection efficiency.

In some embodiments, the polymer of formula (I) used in the invention may be characterized by its molar mass, molar mass distribution (dispersity), the type of amino group (primary or secondary amine) by whether R₁ represents a hydrogen atom or an alkyl group having from 1 to 20 carbon atoms and/or by the percentage of amides along the chain (such as by the ratio m:(n+m)). In the Examples of the present application, the influence of these criteria on the transfection efficiency is demonstrated. In order to prove the efficiency of the polymers of formula (I), linear polyethylene imine (1PEI) was used as reference for the transfection efficiency and the viability.

In some embodiments, the molar mass of the polymer of formula (I) may be from about 9 kg/mol to about 67 kg/mol. In some embodiments, the dispersity of the polymer of formula (I) may be from about 1 to about 2.

In some embodiments, the polyplex according to the invention may comprise a polymer which is obtainable by the following method steps and at least one nucleic acid. The method steps comprise a first step of synthesis of a polyvinylamide and a second step of partial hydrolysis of some of the amide functions. In the first step, a corresponding polyvinylamide may be synthesized as a precursor by any suitable polymerization technique. For example, free radical polymerization (FRP) or by a controlled radical polymerization, such as for example organometallic mediated radical polymerization (OMRP) or polymerization controlled by a reversible addition fragmentation transfer (RAFT) mechanism may be used. In the second step, the subsequent hydrolysis of the amide functions may be an acidic hydrolysis and may optionally be performed with a pre-selected hydrolysis level, for example a hydrolysis level of 70 to 97%, such that a polymer of formula (I) was obtained having a pre-selected ratio of n to the sum of n and m, for example a ratio of n:(n+m) of from 0.7:1 to 0.95:1. In this way, a polymer of formula (I) may be prepared with various molar masses and low or high dispersity, for example a series of PVAAm and PAVAAm polymers containing from 10% to 30% of residual amide groups (such that the ratio m:(n+m) was from 0.1 to 0.3), respectively. In some embodiments, the polymer of formula (I) may be a polymer which is obtainable by the method steps defined above.

As shown in the Examples below, the polymers of formula (I) were used to form polyplexes according to the invention which were tested for pDNA transfection on HeLa cells, and then the most efficient polyplexes were tested for transfection on other cells as well. Depending on the nature of the polymer of formula (I) used, important differences in transfection efficiency were noticed. Generally, for PAVAAm (secondary amines) efficient transfection was obtained and it was in the same order of magnitude as the 1PEI reference or even better.

In the Examples, different polyvinylamines having an amide group (PVAAm) and polyalkylvinylamines having an amide group (PAVAAm) were synthesized by free radical polymerization (FRP) or organometallic-mediated controlled radical polymerization (OMRP) of N-vinylacetamide (NVA) or N-alkylvinylacetamide (NAVA), followed by the acidic hydrolysis of the amide functions. The OMRP technique, as with other controlled polymerization techniques, allowed substantially perfect control of the molar mass of the polymer of formula (I) (from small chains to long chains) and low dispersity. Once the polyvinylamides were obtained, acidic hydrolysis was performed. PNVAs were hydrolyzed using quite soft conditions (HCl 2N at 120°C for 14h). The conversion into a polyvinylamine comprising an amide group was determined by elementary analysis using the C/N ratio in the polymer before and after acidic hydrolysis treatment. In some embodiments, a high hydrolysis level of 87 to 94% was determined, *i.e.* 87-94% of the functions along the chain were primary amines. The secondary polyvinylamides (PNAVA) were more difficult to hydrolyze so that harsher conditions were used according to a formerly established protocol (HCl 6N at 120°C for 64h or HCl 3N at 100°C for several days. Thanks to these conditions, PNAVAs could be hydrolyzed by from 70 to 97%, *i.e.* PAVAAms containing 3 to 30% of amide units along their chains were obtained. Particular precautions were taken in order to avoid the toxicity of the polymers.

### Polyplexes formation and their characterization

In some embodiments, the method for preparing a polyplex comprises combining at least one nucleic acid with at least one polymer of formula (I) in a buffer, for example HEPES buffer. Without wishing to be limited to a particular theory, it is believed that the formation of polyplexes in 10 mM HEPES buffer, pH 7.4 is based on electrostatic interactions between positive charges present in the polymer chain of the at least one polymer of formula (I), thanks to the primary or secondary amines, and the phosphate negative charges of the nucleic acid (for example plasmid DNA, abbreviated pDNA, may be used). In the Examples, the amount of the at least one polymer of formula (I) necessary to allow complete pDNA condensation was determined by agarose gel retardation assay upon electrophoresis of polyplexes made with increasing amounts of the at least one polymer of formula (I). In contrast to free pDNA, pDNA complexed with the at least one polymer of formula (I) did not migrate in the agarose gel upon application of a positive current. Generally, the fluorescence intensity decreased as pDNA condensation increased.

In some embodiments, the polyplexes may have an at least one nucleic acid to at least one polymer of formula (I) weight ratio (WR) suitable to allow nucleic acid condensation, for example a weight ratio of from 1:1 to 1:8. In some embodiments, the polyplexes may have a defined phosphate : nitrogen (P/N) molar ratio, which reveals the number of amines necessary for one phosphate of the at least one nucleic acid. In some embodiments, the polyplex may have a P/N molar ratio which is less than 1:1, for example from 1:2 to 1:30. Interestingly, it was found that less secondary amines functions were needed to condense pDNA with PAVAAm (P/N molar ratio ranging from 1:4 to 1:12) compared to primary amines composing PVAAm (P/N molar ratio ranging from 1:6 to 1:20).

In some embodiments, the polyplex according to the invention may have a small size, for example less than 1 µm, more advantageously less than 500 nm. In some embodiments, the polyplex may have a size from 50 to 300 nm. In some embodiments, the polyplex has a pre-selected size for optimal use in a particular application. Measurements of the polyplex size can be achieved by a number of techniques including, but not limited to, dynamic laser light scattering (photon correlation spectrocopy, PCS), as well as other techniques known to those skilled in the art (see, Washington, Particle Size Analysis in Pharmaceutics and other Industries, Ellis Horwood, New York, 1992, 135-169). In some embodiments, the mean hydrodynamic diameter is from 150, to 300, for example to 260 nm.

In some embodiments, polyplexes were positively charged with a surface potential (ζ) of from + 15, for example from +26 to + 55 mV. It was found that a small size and a positive charge are advantageous for an efficient polyplex. It is believed that positively charged polyplexes interact favorably with the negatively charged cell membrane through electrostatic interactions, as well as their uptake by the cells. Neutral polyplexes with ζ=-1 or -2 mV which were formed in the former study by Wolfert et al. were found to give poor results. The surface charge or potential may be measured by determining the zeta potential of a suspension of the polyplex according to the invention. A sample of the polyplex in a buffer or carrier is injected into a disposable cell and a measurement of the particle electrophoretic mobility results in the calculated zeta potential.

### Transfection efficiency

It was surprisingly observed that high transfections levels (10⁸-10⁹ RLU/mg) were obtained for all PAVAAm polymers independently of the molar mass and the synthesis route (FRP or OMRP), whereas linear influence of the chain length was observed for PVAAm. It was found that decreasing the molar mass of PVAAm by a factor of 10 increased the transfection efficiency by four orders of magnitude, approaching 1PEI efficiency (see compound 1 in Example 1). In some embodiments, a polymer of formula (I) which comprises one set of repeating units wherein R₁ represents a hydrogen atom may have a small molar mass. In some embodiments, the molar mass of a polymer of formula (I) which comprises one set of repeating units wherein R₁ represents a hydrogen atom should be may be from 1000g/mol to 50 000 g/mol, for example to 10000 g/mol. In contrast, it was found that the dispersity, also called molar mass distribution, had no apparent impact on transfection.

### Cytotoxity

A further important factor that influences whether a polymer may be a potential gene carrier is the cytotoxicity, also referred to as cell viability. For the primary polyvinylamines PVAAm, the shortest and most efficient polymers had also the lowest toxicity. For the secondary polyvinylamines polyplexes (PAVAAm), the viability of HeLa cells was better than with 1PEI. It could be observed that the increase of the number of residual amides, *i.e.* the decrease of the density of charges, improves viability.

On other cell lines, with 1PEI the viability was 20% at best, contrary to PAVAAms polyplexes for which 60 to 90% of viability was reached.

In some embodiments, the polymer of formula (I) used in the invention may be in cationic form. In some embodiments, a cationic form of a polymer of formula (I) comprises a protonated amino group and/or a quaternary ammonium group having an optionally substituted straight or branched chain alkyl group of from 1 to 8 carbon atoms bound to an amino group, for example a methyl, ethyl or -CH₂CH₂OH group. In some embodiments, a cationic form of a polymer of formula (I) comprises a biologically acceptable anion such as, for example, trifluoroacetate, monoethylsulfate, acetate, iodide, chloride and/or bromide.

In some embodiments, the polyplex according to the invention comprises at least one nucleic acid comprising a DNA and/or RNA fragment and/or a single or double-stranded, linear or circular, natural or synthetic, modified or unmodified fragment or a portion of a nucleic acid, without size limitation. In some embodiments, the at least one nucleic acid may be for example a genomic DNA, a cDNA, a mRNA, an antisense RNA, a ribosomal RNA, a ribozyme, a tRNA, DNA encoding such an RNA and/or an analog such as a morpholino. Herein it should be understood that "polynucleotide" and "nucleic acid" are synonyms. In some embodiments, the at least one nucleic acid may also be in the form of a plasmid or linear polynucleotide which contains at least one coding sequence of nucleic acid that can be transcribed and translated to generate a polypeptide, ribozyme, antisense or other molecule of interest upon delivery to a cell. In some embodiments, the at least one nucleic acid may be an oligonucleotide which is to be delivered to the cell, e.g., for an antisense or ribozyme function. In some embodiments, the at least one nucleic acid may be co-formulated with a viral proteins, a cationic lipid, and/or a cationic polymer.

In some embodiments, the at least one nucleic acid may comprise DNA and RNA which forms a polypeptide of interest in a cell which may stay within the cell or which may be secreted from the cell. In some embodiments, the at least one nucleic acid is a plasmid DNA which may direct the synthesis of relatively large amounts of an encoded polypeptide. When the at least one nucleic acid delivered to a cell encodes an immunizing polypeptide, for example, the polyplex according to the invention can be applied to achieve improved and effective immunity against infectious agents, including intracellular viruses, and also against tumor cells. In some embodiments, the at least one nucleic acid comprises genetic information necessary for expression by a target cell such that it comprises all the elements required for transcription of said DNA into mRNA and for translation of mRNA into a polypeptide. In some embodiments, the at least one nucleic acid comprises a transcriptional promoters suitable for use in a vertebrate system. For example, a suitable promoter includes a viral promoter RSV, MPSV, SV40, CMV or 7.5k vaccinia promoter, and/or an inducible promoter. In some embodiments, the at least one nucleic acid may also include an intron sequence, a targeting sequence, a transport sequence and/or a sequence involved in replication or integration. These sequences have been reported in the literature and can be readily obtained by a person of skill in the art. In some embodiments, the at least one nucleic acid may be stabilized with a specific component such as protamine or spermine.

In some embodiments, the at least one nucleic acid may be homologous or heterologous to the expressing target cell. In some embodiments, the at least one nucleic acid encodes all or part of a polypeptide, particularly a therapeutic or prophylactic polypeptide. A polypeptide should be understood to be any translational product of a polynucleotide regardless of size, and whether glycosylated or not, and includes peptides and proteins. It should be understood herein that a therapeutic polypeptide includes a polypeptide which can compensate for a defective or deficient protein in a human or an animal; a polypeptide which can act through a toxic effect to limit or remove a harmful cell from a human or animal body; and/or an immunity-conferring polypeptide which may act as an endogenous immunogen to provoke a humoral and/or cellular response. Examples of polypeptides which may be encoded by the at least one nucleic acid used in the invention include an antibody, enzyme, hormone, cytokine, membrane receptor, structural polypeptide, transport polypeptide, adhesin, ligand, transcription factor, transduction factor, replication factor, stabilisation factor, antibody, more particularly CFTR, dystrophin, factors VIII or IX, E6 or E7 from HPV, MUC1, BRCA1, interferon β, interferon γ, interleukin IL-2, IL-4, IL-6, IL-7, IL-12, GM-CSF (Granulocyte Macrophage Colony Stimulating Factor), tk gene from Herpes Simplex type 1 virus (HSV-1), p53, VEGF, tryptophan. It should be understood that herein an antibody encompasses a whole immunoglobulin of any class, a chimeric antibody and/or a hybrid antibody with dual or multiple antigen or epitope specificities, and/or a fragment thereof, such as F(ab)2, Fab', Fab including a hybrid fragment and/or an anti-idiotype.

In some embodiments, the at least one nucleic acid used in the invention may have any of the properties or functions which the nucleic acid represented by SEQ ID NO: 1 has, for example a pCMV-EGFP plasmid encoding the enhanced green fluorescent protein from yellowfish (EGFP) gene (SEQ ID NO:2) under the control of the Cytomegalovirus CMV promoter. In some embodiments, the at least one nucleic acid used in the invention may have any of the properties or functions which the nucleic acid represented by SEQ ID NO:3 has, for example a pTG11033 plasmid encoding the luciferase gene (SEQ ID NO: 4) placed under the control of the CMV promoter, the HMG gene intron 1 and the SV40 polyA termination signal.

In some embodiments, the at least one nucleic acid used in the invention may comprise the sequence of SEQ ID NO:1, SEQ ID NO:2, SEQ ID NO:3 or SEQ ID NO:4 or a homologue of SEQ ID NO:1, SEQ ID NO:2, SEQ ID NO:3 or SEQ ID NO:4, or a fragment of SEQ ID NO:1, SEQ ID NO:2, SEQ ID NO:3 or SEQ ID NO:4 or of said homologue. Such a fragment may have a length of at least 5000, 1000, 500, 300 or 100 nucleotide bases.

A homologue typically has at least 70% identity to the original nucleic acid (in this case SEQ ID NO:1, SEQ ID NO:2, SEQ ID NO:3 or SEQ ID NO:4) or at least 80% or 90%, and more preferably at least 95%, 97% or 99% identity thereto over at least 20, preferably at least 30, for instance at least 40, 60, 100, 1000 or more contiguous bases.

Homology can be measured using known methods. For example the UWGCG Package provides the BESTFIT program which can be used to calculate homology (for example used on its default settings) (Devereux et al (1984) Nucleic Acids Research 12, p387-395). The PILEUP and BLAST algorithms can be used to calculate homology or line up sequences (typically on their default settings), for example as described in Altschul S. F. (1993) J Mol Evol 36: 290-300; Altschul, S, F et al (1990) J Mol Biol 215:403-10. Software for performing BLAST analyses is publicly available through the National Center for Biotechnology Information (http://www. ncbi. nlm. nih. ov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul et al, supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands. The BLAST algorithm performs a statistical analysis of the similarity between two sequences; see e. g., Karlin and Altschul (1993) Proc. Natl. Acad. Sci. USA 90: 5873-7. One measure of similarity provided by the BLAST algorithm is the smallest sum probability (P (N)), which provides an indication of the probability by which a match between two nucleotide sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

Typically the homologous sequence differs from SEQ ID NO:1, SEQ ID NO:2, SEQ ID NO:3 or SEQ ID NO:4 by less than 1000, 500, 100 or 50 mutations (substitutions, insertions or deletions). In one embodiment the homologous sequence differs from SEQ ID NO:1, SEQ ID NO:2, SEQ ID NO:3 or SEQ ID NO:4 by less than 10, 20 or 30 nucleotide bases over any stretch of 100 contiguous nucleotides of SEQ ID NO:1, SEQ ID NO:2, SEQ ID NO:3 or SEQ ID NO:4.

In some embodiments, the at least one adjuvant used in the pharmaceutical composition according to the invention may be a positively or negatively-charged, neutral or zwitterionic lipid, a protic polar compound and/or an aprotic polar compound. An example of a suitable protic polar compound includes propylene glycol, polyethylene glycol, glycerol, EtOH, 1-methyl L -2-pyrrolidone and/or their derivative. An example of a suitable aprotic polar compound includes dimethylsulfoxide (DMSO), diethylsulfoxide, di-n-propylsulfoxide, dimethylsulfone, sulfolane, dimethylformamide, dimethylacetamide, tetramethylurea, acetonitrile and/or their derivatives. For example the adjuvant may be a triglyceride, a diglyceride, cholesterol; in particular a positively or negatively-charged, neutral or zwitterionic lipid which is or derives from a phosphatidylethanolamine (PE), phosphatidylcholine, phosphocholine, dioleoylphosphatidylethanolamine (DOPE), sphingomyelin, ceramide or cerebroside, a cationic lipid such as for example a glycerolipid or PEGylated lipid or a lysomotropic agent such as chloroquine. In some embodiments, the at least one adjuvant may be selected to improve formation of the polyplex, to facilitate transport of the polyplex across a cell membrane and/or to prevent endosomal degradation.

In some embodiments, the weight ratio of polymer of formula (I) to adjuvant may be from 1:0.1 to 1:10, depending of the type of adjuvant. In some embodiments, the weight ratio is 1:1. A skilled person would be capable of selecting a suitable weight ratio. In some embodiments, the adjuvant comprises a mixture of positively or negatively-charged, neutral and/or zwitterionic lipids.

In some embodiments, a cell used in the method of transferring according to the invention may be a prokaryote cell, eukaryote cell, yeast cell, plant cell, human cell or animal cell, in particular a mammalian cell. In some embodiments, a cell may be a cancer cell. In some embodiments, the method of transferring according to the invention may be applied in vivo to an interstitial or luminal space of tissue in lungs, trachea, skin, muscles, brain, liver, heart, spleen, bone marrow, thymus, bladder, lymphatic system, blood, pancreas, stomach, kidneys, ovaries, testicles, rectum, peripheral or central nervous system, eyes, lymphoid organs, cartilage, and/or endothelium. In some embodiments, the cell may be a muscle cell, a hematopoietic system stem cell or an airways cell, for example a tracheal or pulmonary cell, especially a cell of the respiratory epithelium.

In some embodiments, the method of transferring according to the present invention comprises contacting a cell with at least one polyplex or composition according to the invention. This method may be applied by direct administration of said polyplex or composition to cells of the human or animal in vivo, or by in vitro treatment of cells which were recovered from the human or animal and which are then re-introduced into the human or animal body in an ex vivo process. In in vitro application, cells cultivated on an appropriate medium are placed in contact with a suspension comprising a polyplex or composition of the invention. After an incubation time, the cells are washed and recovered. Introduction of the at least one nucleic acid can be verified (eventually after lysis of the cells) by any appropriate method.

In some embodiments, where the transferring method according to the invention comprises an in vivo treatment, the patient may undergo a macrophage depletion treatment prior to administration of the pharmaceutical composition according to the invention in order to improve the transfection rate. Such a technique is described in the literature (see Van Rooijen et al., 1997, TibTech, 15, 178-184).

The pharmaceutical composition may be administered in a number of ways such as for example by topical, cutaneous, oral or parenteral administration. Parenteral administration includes intravenous, intraarterial, subcutaneous, intraperitoneal or intramuscular injection.

The pharmaceutical composition for topical administration may include transdermal patches, ointments, lotions, creams, gels, drops, sprays, liquid and powders. Conventional pharmaceutical carriers, aqueous, powder or oily bases, thickeners and the like may be necessary or desirable.

The pharmaceutical composition preferably contains a vehicle which is pharmaceutically acceptable for an injectable formulation, in particular for direct injection into the desired organ. The injection may be a direct injection into the tissues or the circulatory pathways or a treatment of cells in culture followed by their reimplantation in vivo, by injection or graft. The composition may be in solid, liquid, semiliquid or aerosol form, in an organic solution or aqueous dispersion.

The doses of nucleic acid used for the injection and the number of administrations may be adapted according to various parameters, such as to the mode of administration used, the gene to be expressed, the disease to be cured and the duration of the treatment.

The present invention provides an effective method for delivering DNA into cells for gene therapy or DNA based vaccination or other therapeutic applications.

The pharmaceutical composition according to the invention may be administered by intravenous injection such as for example in gene therapy for transfection of hepathocyte in liver aiming to introduce a gene a) for substitution as in the case of familial type IIa hypercholesterolemia or ornithine carbamyl transferase or type VII mucopolysaccharidosis (deficit of ß-glucuronidase) or Sly disease, or b) to produce factor IX by hepatocytes for the treatment of haemophilia B. The polymer of formula (I) could be manufactured as a powder to solubilize to make a polyplex with a plasmid DNA encoding the factor IX or a polyplex could be provided as a solution or a lyophilizate of a polyplex containing the plasmid DNA encoding factor IX. A similar approach may be done with a plasmid encoding a gene encoding a toxic protein (suicide gene) to kill cancer cells or siRNAs directed against the mRNA of inhibitor of apoptosis for the treatment of certain cancers and metastases.

The pharmaceutical composition according to the invention may further be administered by aerosolisation such as for example in gene therapy for cystic fibrosis using a plasmid DNA encoding the CFTR gene. The polyplex could be provided as a solution or a lyophilizate of a polyplex to be nebulized.

The pharmaceutical composition according to the invention may further be administered through intramuscular, intradermal and subcutaneous injection such as for example in RNA vaccines. The polymer of formula (I) could be manufactured as a powder to solubilize to make a polyplex with messenger RNA coding for tumor or viral antigens.

The invention is illustrated by the following Figures of the accompanying drawings in which:
**Figure 1** represents the transfection efficiency of HeLa cells performed with PVAAm and PMVAAm polyplexes formed from compounds 1 to 5 and 1PEI at two pDNA/polymer ratios as described in Examples 1, 2 and 3;
**Figure 2** represents the cell viability of HeLa cells after transfection performed with PVAAm and PMVAAm polyplexes formed from compounds 1 to 5 and 1PEI at two pDNA/polymer ratios as described in Examples 1, 2 and 3;
**Figure 3** represents the transfection efficiency of HeLa cells performed with P(VAAm-co-MVAAm) polyplexes at two pDNA/polymer ratios as described in Example 4;
**Figure 4** represents the cell viability of HeLa cells after transfection performed with P(VAAm-co-MVAAm) polyplexes at two pDNA/polymer ratios as described in Example 4;
**Figure** 5 represents the transfection efficiency of HeLa cells performed with P(MVAAm-co-VIm) and P(VAAm-co-MVAAm-co-VIm) polyplexes at two pDNA/polymer ratios as described in Example 5;
**Figure 6** represents the cell viability of HeLa cells after transfection performed with P(MVAAm-co-VIm) and P(VAAm-co-MVAAm-co-VIm) polyplexes at two pDNA/polymer ratios as described in Example 5;
**Figure 7** represents the transfection efficiency of H2K2B4, C2C12, 16HBE and DC2.4 cells performed with different polyplexes at two pDNA/polymer ratios as described in Example 2;
**Figure 8** represents the cell viability of H2K2B4, C2C12, 16HBE and DC2.4 cells after transfection performed with different polyplexes at two pDNA/polymer ratios as described in Example 2;
**Figure 9** represents the transfection efficiency of polyplexes comprising no amide units prepared as described in Comparative Example 1; and
**Figure 10** shows fluorescent images (left), microscopy images (middle) and superposition (right) analysis of EGFP positive cells, respectively HeLa, C2C12, DC2.4 and Fibroblast, transfected by polyplexes of compound 2 at P/N ratio = 1/3 (A) and P/N ratio = 1/6 (B).

The invention will now be illustrated by the following examples which are not intended to limit the scope of the claims.

### EXAMPLE 1

### Synthesis of PNVA by controlled radical polymerization

A first polymer of formula (I) in the form of a polyvinylamine containing residual amide functions was synthesized according to [3] and is referred to as compound 1. The contents of reference [3] are incorporated herein by reference, particularly where it concerns synthesis of a polymer. Using an organometallic-mediated controlled radical polymerization (OMRP) allowed to synthesize polymers with various molar masses and narrow molar mass distributions (dispersity). *N*-vinylacetamide (NVA) (> 98%, TCI), cobalt(II) acetylacetonate (Co(acac)2) (97%, Aldrich), 2,2'-azobis[2-methyl-*N*-(2-hydroxyethyl)propioamide] (VA-086, t½ = 10h at 86°C) (> 98%, Wako) were used as received and added in a reactor designed for photochemistry (Laboratory-UV-Reactor system 2 purchased from UV-Consulting Peschl). The molar ratio [NVA]/[VA-86]/[Co(acac)₂] used for compound 1 was 110/2/1. The medium is degassed by three vacuum/argon cycles before addition of degassed MeOH (15 mL). The reactor was maintained at 0°C in an ice-bath and the mixture was irradiated with an UV lamp (TQ 150 with a power input at 150 4 W) for 4h at 0°C. After irradiation was stopped, the medium was allowed to return to room temperature (∼20°C) and the polymerization proceeded for 4 hours. A sample was picked out of the reactor, quenched by TEMPO, and used for determining the conversion by 1H NMR (23%) and the molar mass of the polymer by SEC analyses. In order to quench the reaction, degassed 1-propanethiol (1.4 mL, 15.8 mmol) was added with a syringe under inert atmosphere to the reaction medium which became instantaneously dark black. The medium was stirred overnight at room temperature. The reaction medium was eluted trough a celite allowing removing black residues. The polymer was then recovered by several precipitations in acetone and dried under vacuum. The polymer was further purified by dialysis against water for two days. After lyophilisation, an off-white solid was obtained and characterized by SEC, NMR and ICP.

The second step was the hydrolysis. The polymer was dissolved in hydrochloric acid (HCl, 2N). The resulting solution of polymer (5% w/v) was then placed in an Acid Digestion Parr Vessel and heated at 120°C for 14h. After cooling, the solution was dialyzed against pure distilled water for one night. During the last two hours of dialysis, the pH was maintained at 7. The polymer was recovered upon lyophilization and was analyzed by NMR. The determination of the hydrolysis rate for each polymer was achieved using the elementary analysis. The method was based on the C/N ratio in the polymer before and after acidic treatment rather than on absolute mass percentage of C and N in order to prevent mistakes coming from the hydroscopic character of the polymer. Results are presented in Table 1.

**TABLE 1:**

| **CHARACTERISTICS OF POLYMER USED IN EXAMPLE 1, BEFORE AND AFTER HYDROLYSIS** | | | | | | | |
|---|---|---|---|---|---|---|---|
| | | Polymers before hydrolysis | | Polymers after hydrolysis | | | |
| Compound | Reference | Monomer | DP | % hydrolysis | n | m | Mₙ kg/mol |
| 1 | PN-C150 | N-Vinylacetamide | 139 | 91 | 126 | 13 | 6.0 |

### Preparation of the polyplexes

Once the polymer had been obtained, the next step was the preparation of the polyplexes. Therefore, plasmid DNA was used. pCMV-luc (pTG11033 having SEQ ID NO:3, 9514 bp, Transgene S.A., Strasbourg, France) was a plasmid DNA (pDNA) encoding the firefly luciferase (luc) gene (SEQ ID NO:4) under the control of the human cytomegalovirus promoter. pCMV-EGFP (5130 bp) was homemade plasmid DNA having SEQ ID NO: 1 encoding the jellyfish Aequorea victoria enhanced green fluorescent protein (EGFP) (SEQ ID NO:2) under the control of the human cytomegalovirus. Supercoiled plasmid was isolated from Escherichia coli DH5a super competent bacteria (Invitrogen, Cergy Pontoise, France) by alkali lysis and purification with QIAGEN Mega Kit Endotoxin free Plasmid (Qiagen, Courta-boeuf, France).

Polyplexes were prepared at various DNA/polymer weight ratios (WR) in HEPES 10 mM at pH 7.4. The required amount of polymers in 30 µL HEPES was added to pDNA solution (2.5 µg in 33 µL of HEPES) and vortexed for 4s. After 30 min at room temperature, the solutions containing polyplexes were adjusted to a total volume of 500 µL using the medium supplemented without FBS (MEM for HeLa and DMEM for the other cell types). The size of polyplexes measured in HEPES 10 mM at pH 7.4 by dynamic light scattering and ζ-potential measurements were performed in HEPES 10 mM at pH 7.4 by using SZ-100 Nanopartica (Horiba, Les Ulis, France). Samples were illuminated with a 633 nm laser, and the intensities of scattered light at an angle of 173° were measured using an avalanche photodiode. The z-average hydrodynamic diameter (Dz), dispersity and distribution profiles of the samples were automatically calculated. Results are presented in Table 2.

**TABLE 2: CHARACTERISTICS OF POLYPLEX FORMED IN EXAMPLE 1**

| | Polyplexes | | | | |
|---|---|---|---|---|---|
| Compound | Ratio | pDNA/polymer weight ratio | Phosphate/amine molar ratio | Hydrodynamic diameter (nm) | ζ potential (mV) |
| 1 | 1 | 1/4 | 1/25 | 140 | +50 |
| 1 | 2 | 1/8 | 1/50 | 190 | +42 |

### Gel retardation assay

Polyplexes were freshly prepared at various weight ratios in a solution of HEPES 10 mM pH 7.4. Samples of 20 µL were loaded onto 0.9% agarose gel containing EtBr (0.3 mg/mL) in a Tris-borate-EDTA buffer, pH 8.6 (95 mM Tris, 89 mM boric acid, 2.5 mM EDTA and 10 mM DTT, pH 8.6) and ran at 100 V for 45 min. The gels were analyzed using the Azure™Biosystem c600 to visualize pDNA.

### Cell culture

Human epithelioid cervix carcinoma cells (HeLa cells; CRL1772, C2C12, Rockville, MD, USA) were cultured in MEM medium supplemented with 10% FBS, 100 U/mL penicillin, 100 mg/mL streptomycin, 1% non-essential amino acid, and 1% GlutaMAX™. Cells were maintained at 37°C in a humidified 5% CO₂ atmosphere. Cells were checked for mycoplasma presence using the MycoAlert1 Mycoplasma Detection Kit (Lonza, Levallois Perret, France).

### Transfection and luciferase gene expression measurement

One day before transfection, cells were seeded in a 24-well plate at a density of 1.5 x 10⁵ cells per well in 1 mL in culture medium. They were incubated for 4h at 37°C with the freshly made polyplexes (500 µL, 2.5 µg DNA). Then, the medium was removed and replaced with fresh complete medium. The Luciferase activity was measured 48h after transfection by using a luminometer (LUMAT LB 9507). Then the Luciferase activity was normalized to total cell protein using a BCA protein assay kit (Uptima, Interchim SA, Montluçon, France), and expressed as relative light units (RLU) per mg of protein. Data are presented as means and standard deviation (s.d.) based on three independent experiments, each in duplicate for the determination of the RLU and in triplicate for protein quantification.

### Transfection efficiency of PVAAm on HeLa cells

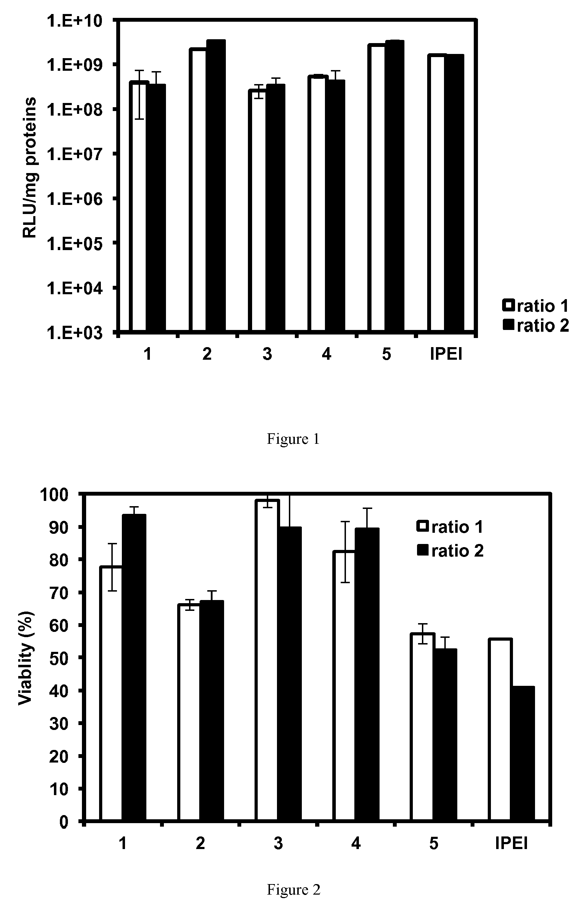

Transfection efficiency and cytotoxicity of polyplexes were evaluated at two P/N ratios on HeLa cells. LPEI (512 kg/mol) was used as reference. Transfection was carried out with pDNA (pTG11033) encoding the luciferase gene and the luciferase activity was evaluated 48h post-transfection. Results are presented in Fig. 1.

Compound 1, with a short molar mass, showed efficient transfection. Interestingly, transfection was close to that obtained with 1PEI (10⁸ *vs*. 10⁹ RLU/mg proteins).

### Cytotoxicity

An MTT assay was performed to determine the toxicity of polyplexes and free polycations. 48 h after transfection, cells were washed twice with PBS and then 50 µL of MTT (3-(4,5-dimethylthiazol-2-yl)-2,5-diphenyltetrazolium bromide; 5 mg/ml in PBS) was added to each well and the cells were incubated for 4 h at 37°C. The medium was taken of and the cells were washed with PBS (500 µL). Then acidified isopropanol (1 mL) and a solution at SDS 3% (200 µl) were added. MTT was thus converted in water-soluble formazan. The absorbance was measured at 560 nm with a Victor I spectrophotometer and expressed as a percentage of the absorbance of untransfected cells. Data are presented as mean and standard deviation based on two independent experiments, each in triplicate. Results are presented in Fig.2.

The cell viability in the presence of compound 1 was excellent (up to 95%).

### EXAMPLE 2

### Synthesis of PNMVA by free radical polymerization

A second series of polymers of formula (I) referred to as compounds 2 and 3 (PMVAAm) were synthesized by free radical polymerization (FRP) according to [3] (*Polym*. *Chem*., 2016, 7, 69-78). *N*-methylvinylacetamide (NMVA) (> 98% Aldrich) was purified by distillation under reduced pressure and degassed by freeze-drying cycles under vacuum. 2,2'-azobis(4-methoxy-2,4-dimethyl valeronitrile) (V70) was used as received and placed under argon in a flask, dissolved in degassed methanol (14 mL) followed by addition with a syringe of distilled and degassed NMVA (14 mL, 13.4 g, 135 mmol). The reaction mixture was then heated at 30°C for 4h. The polymer was then purified by precipitation in diethyl ether, filtrated and dried under vacuum before dissolution in water and dialysis against water for two days. After lyophilization, PNMVA was collected as a white solid and characterized by 1H NMR in D₂O. The ratio [NMVA]/[V70] was 100 : 1 for the two compounds. Compound 3 differs from compound 2 in that the reaction time was extended to 22 hours such that it has a higher degree of polymerisation.

Once the polyvinylamides were obtained, acidic hydrolysis was performed as described in example 1, except that harsher conditions were used: HCl 6N at 120°C for 64h. Results are presented in Table 3.

**TABLE 3: CHARACTERISTICS OF POLYMERS USED IN EXAMPLE 2, BEFORE AND AFTER HYDROLYSIS**

| | | Polymers before hydrolysis | | Polymers after hydrolysis | | | |
|---|---|---|---|---|---|---|---|
| Compound | Reference | Monomer composition | DP | % hydrolysis | n | m | Mₙ kg/mol |
| 2 | PM-F140 | N-Methylvinylacetamide | 138 | 81 | 112 | 26 | 9.0 |
| 3 | PM-F285 | N-Methylvinylacetamide | 284 | 71 | 202 | 82 | 10.2 |

After hydrolysis, polyplexes were formed as described in example 1. Results are presented in Table 4. The same conditions for cell culture were used as in example 1.

**TABLE 4: CHARACTERISTICS OF POLYPLEXES FORMED IN EXAMPLE 2**

| | Polyplexes | | | | |
|---|---|---|---|---|---|
| Compound | Ratio | pDNA/polymer weight ratio | Phosphate/amine molar ratio | Hydrodynamic diameter (nm) | ζ potential (mV) |
| 2 | 1 | 1/3 | 1/12 | 230 | +42 |
| 2 | 2 | 1/6 | 1/24 | n.d. | n.d. |
| 3 | 1 | 1/2 | 1/7 | 130 | +32 |
| 3 | 2 | 1/4 | 1/13 | 150 | +33 |

### Transfection efficiency and cytotoxicity of PMVAAm polyplexes on HeLa cells

As in example 1, transfection efficiency and cytotoxicity of polyplexes were evaluated at two P/N ratios on HeLa cells. Results are presented in Fig. 1. Compounds 2 and 3 showed efficient transfection. Contrary to PVAAm polyplexes, no significant influence of the polymer chain length was noticed. Depending on weight ratio, a high level of luciferase activity (10⁸ to 10⁹ RLU/mg proteins) was observed with all compounds. The efficiency of PMVAAm was comparable to and even slightly higher than for 1PEI.

The cell viability is presented in Fig. 2. The viability obtained with compounds 2 and 3 was better than with the reference polymer, 1PEI. Indeed, a viability of the cells ranging from nearly 60 to 70 % was measured whereas viability measured for 1PEI samples was no more than 50%.

### Transfection efficiency and cytotoxicity of PMVAAm polyplexes on other cell lines

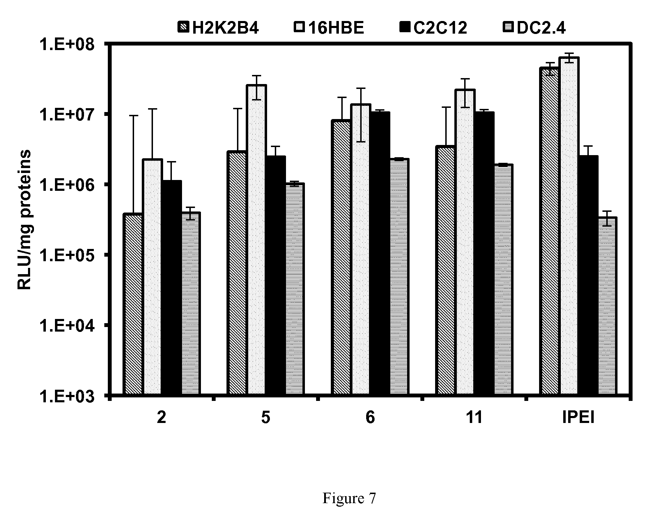

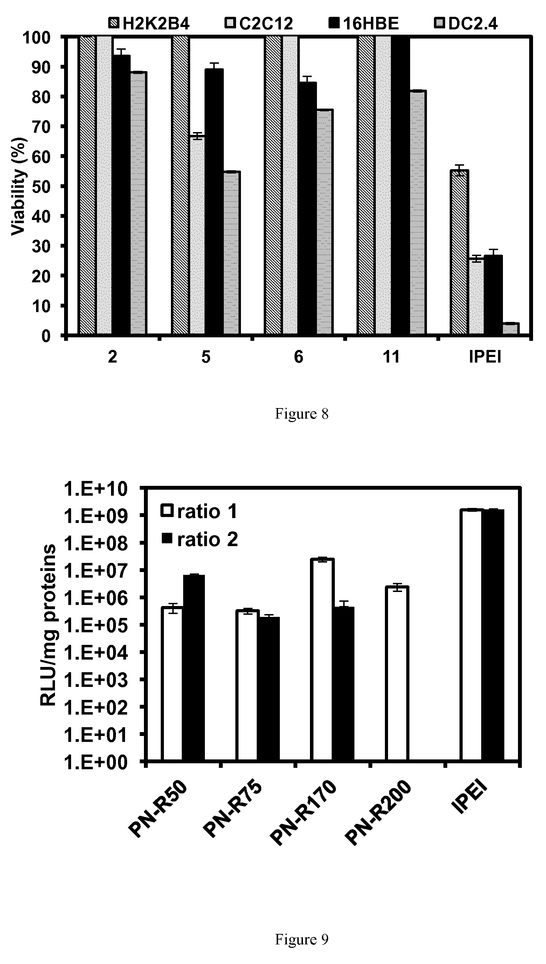

The efficiency and the cytotoxicity of compound 2 was evaluated on other cells lines, namely muscular (H2K2B4 and C2C12), pulmonary (16HBE) and dendritic (DC2.4) cells known to be difficult to transfect. Results are presented in Fig. 7 and Fig. 8 respectively. On pulmonary and dendritic cells, the luciferase activity in cells transfected with PMVAAm polyplexes was close to that obtained with 1PEI polyplexes (∼10⁶-10⁷ RLU/mg proteins). In contrast, on muscular cells the transfection efficiency was about two orders below 1PEI (of 10⁶ *vs*.10⁸ RLU/mg proteins). The cytotoxicity of 1PEI polyplexes was again high: it was 50%, 30%, 30% and 10% for H2K2B4, C2C12, 16HBE and DC2.4, respectively. In contrast, the viability of all cell lines was between 90% and 100% with compound 2 polyplexes. This polymer prepared by FRP was further used to transfect HeLa, C2C12, DC2.4 cells and fibroblasts with a plasmid DNA encoding EGFP which can be detected thanks to its fluorescence emission.

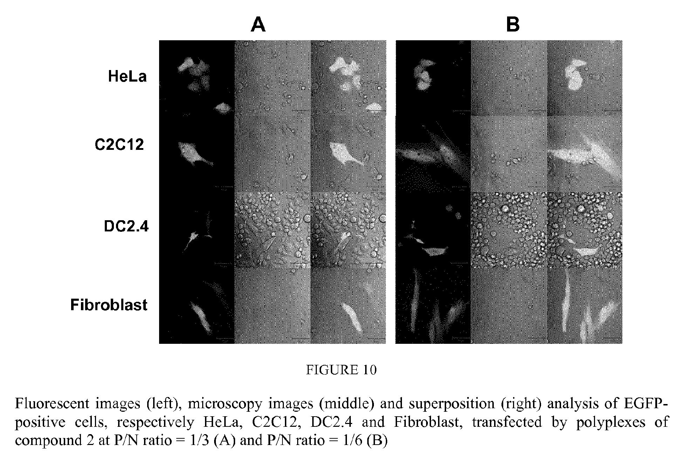

Phase contrast images are presented in Fig. 10. Images presented in the middle indicate whether cells are living or dead. Indeed, when cells enter apoptosis the typical cell's shape changes (here towards spherical or shrinked vesicular structures). The images at the right show the overlay of fluorescence and phase contrast images. For all studied cells lines, EGFP-positive cells preserved their typical cell shape meaning that the cells were transfected without any damage.

Flow cytometry analysis (with instrument LSR, Becton Dickinson, recording at 520 nm after excitation at 488 nm) allowed determination of the number of transfected cells as well as the level of gene expression. The number of transfected cells was similar with compound 2 and 1PEI but the gene expression was higher with the former.

### EXAMPLE 3

### Synthesis of PNMVA by controlled radical polymerization

Another series of polymers of formula (I) were synthesized, from the same monomer as that of example 2 but according to a controlled synthesis, as described in [4] (Debuigne et al., Chemistry - A European Journal 2012, 18, p12834). They are referred to as compounds 4 and 5. A solution of alkyl-cobalt (III) initiator ([Co(acac)2-(CH(OAc)-CH₂)∼4R0] in CH₂Cl₂) was introduced in a flask under argon and evaporated to dryness under reduced pressure at room temperature. Distilled NMVA (6 mL, 58 mmol) was added under argon into the flask and the polymerization mixture was stirred at 40°C. After 16h, the polymerization was stopped by addition of 1-propanethiol (0.350 mL, 53.9 mmol) and the medium became instantaneously dark black. Stirring was maintained overnight at room temperature. After elution of the polymer solution trough a celite pad for removing the black matter, the polymer was precipitated in diethylether, filtered and dried. A second precipitation was performed in Et2O after solubilizing the polymer in MeOH. Finally, the polymer was purified by dialysis against water for two days and was collected as an off-white solid after lyophilisation. The resulting PNMVA was analyzed by SEC and1H NMR. The compounds differ by the [NMVA]/[RCo(acac)2] molar ratio and the reaction time. Compound 4 and 5 have respectively a [NMVA]/[RCo(acac)2] molar ratio of 302 and 297 and the reaction time was respectively 20.5 and 16h. Results are presented in Table 5.

**TABLE 5: CHARACTERISTICS OF POLYMERS USED IN EXAMPLE 3, BEFORE AND AFTER HYDROLYSIS**

| | | Polymers before hydrolysis | | Polymers after hydrolysis | | | |
|---|---|---|---|---|---|---|---|
| Compound | Reference | Monomer composition | DP | % hydrolysis | n | m | Mₙ kg/mol |
| 4 | PM-C265 | N-Methylvinylacetamide | 265 | 87 | 230 | 35 | 16.6 |
| 5 | PM-C310 | N-Methylvinylacetamide | 313 | 93 | 291 | 22 | 18.7 |

Once the polyvinylamides were obtained, acidic hydrolysis was performed as described in example 2. Results are presented in Table 6.

**TABLE 6: CHARACTERISTICS OF POLYPLEXES FORMED IN EXAMPLE 3**

| | Polyplexes | | | | |
|---|---|---|---|---|---|
| Compound | Ratio | pDNA/polymer weight ratio | Phosphate/amine molar ratio | Hydrodynamic diameter (nm) | ζ potential (mV) |
| 4 | 1 | 1/2 | 1/9 | 130 | +47 |
| 4 | 2 | 1/4 | 1/18 | 130 | +47 |
| 5 | 1 | 1/3 | 1/15 | 220 | +32 |
| 5 | 2 | 1/6 | 1/30 | 140 | +42 |

### Transfection efficiency and cytotoxicity

Compounds 4 and 5 both showed high transfection efficiency on HeLa cells (Fig. 1). The efficiency of compound 5 was even higher than the reference 1PEI, showing that polyvinylamines bearing pendant secondary amines with higher molar mass can also be used for nucleic acid transfection. The efficiency and the cytotoxicity of compound 5 were further evaluated on other cells lines as described in example 2 (Fig. 7 and 8). Compound 5 led to the highest number of transfected cells and highest level of EGFP expression as shown by flow cytometry analysis.

### EXAMPLE 4

A series of polymers according to formula (I) in the form of statistical copolyvinylamines containing primary and secondary amines and referred to herein as compounds 6 to 10, were prepared by copolymerization of NVA and NMVA by OMRP or FRP followed by acidic hydrolysis (cfr example 1 and 2 respectively). Two different conditions were tested; 2N HCl/120°C/14h (soft conditions) and 6N HCl/120°C/64h (harsh conditions). With both conditions, residual amide functions remained in the polymer backbone but their quantity varied with the conditions. Soft conditions led to 90% of hydrolysis of NVA and 20-75% of NMVA, and a global rate ranging from 50% to 80%. For harsher conditions, NVA was hydrolyzed up to 94% and NMVA 75% to 90% with a global hydrolysis rate up to 80-90%. The characteristics of the compounds obtained before and after hydrolysis are listed in Table 7. The F_{NVA} and F_{NMVA} values given in Table 7 represent the fractions of the respective monomers in the polymers after hydrolysis. Only compounds obtained by soft conditions are presented in this table.

**TABLE 7: CHARACTERISTICS OF POLYMERS USED IN EXAMPLE 4, BEFORE AND AFTER HYDROLYSIS**

| | | Polymers before hydrolysis | | Polymers after hydrolysis | | | | |
|---|---|---|---|---|---|---|---|---|
| Compound | Reference | Monomers | DP(NVA) - (NMVA) | NVA %hydrol. | NMVA %hydrol. | F_{NVA} | F_{NMVA} | Mₙ kg/mol |
| 6 | P(N114-M114)-C | NVA - NMVA | 114-114 | 90 | 63 | 0.05 | 0.20 | 13.6 |
| 7 | P(N240-M90)-C | NVA - NMVA | 240-90 | 90 | 66 | 0.07 | 0.09 | 17.6 |
| 8 | P(N130-M160)-F | NVA - NMVA | 130-160 | 90 | 78 | 0.04 | 0.12 | 16.8 |
| 9 | P(N40-M127)-C | NVA - NMVA | 40-127 | 90 | 42 | 0.02 | 0.44 | 12.2 |
| 10 | P(N60-M60)-C | NVA - NMVA | 60-60 | 90 | 21 | 0.05 | 0.42 | 8.4 |

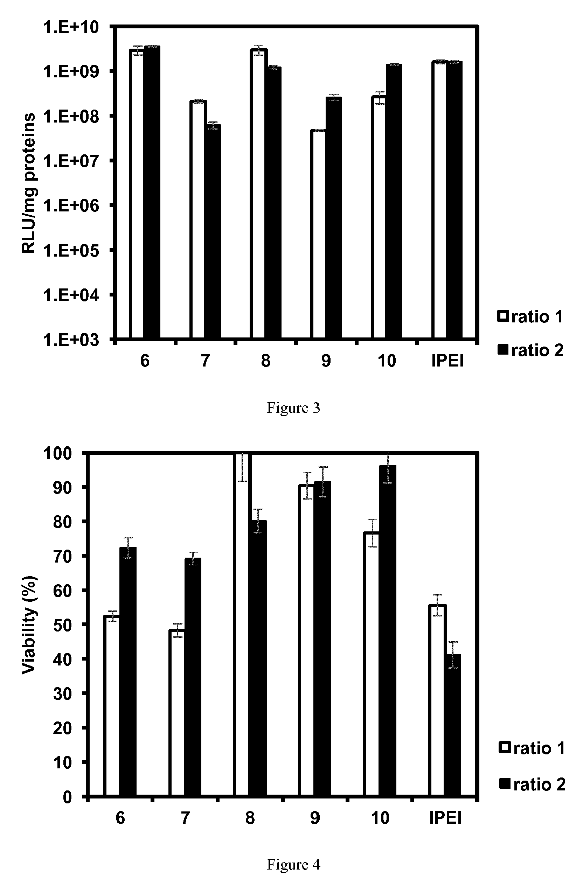

pCMV-luc (pTG11033, 9514 bp) encoding the firefly luciferase was used as plasmid DNA for the determination of the transfection efficiency, whereas pCMV-EGFP (5130 bp) encoding the jellyfish Aequorea Victoria enhanced green fluorescent protein (EGFP) was used as plasmid DNA for FACS analyses. All polyplexes were tested on HeLa cells for transfection efficiency and cell viability. The characteristics of the polyplexes are presented in Table 8 and results in Fig. 3 and 4 respectively.

**TABLE 8: CHARACTERISTICS OF POLYPLEXES FORMED IN EXAMPLE 4**

| | Polyplexes | | | | |
|---|---|---|---|---|---|
| Compound | Ratio | pDNA/polymer weight ratio | Phosphate/amine molar ratio | Hydrodynamic diameter (nm) | ζ potential (mV) |
| 6 | 1 | 1/3 | 1/5 | 290 | +48 |
| 6 | 2 | 1/6 | 1/10 | 5510 | +48 |
| 7 | 1 | 1/1 | 1/1 | 90 | +41 |
| 7 | 2 | 1/3 | 1/3 | 145 | +48 |
| 8 | 1 | 1/1 | 1/1 | 150 | +47 |
| 8 | 2 | 1/3 | 1/4 | 290 | +47 |
| 9 | 1 | 1/2 | n.d. | 110 | +37 |
| 9 | 2 | 1/4 | n.d. | 130 | +40 |
| 10 | 1 | 1/3 | 2/3 | 270 | +50 |
| 10 | 2 | 1/6 | 1/3 | 220 | +48 |

where n.d. means 'not determined'.

The influence of the NMVA amide hydrolysis rate in the copolymers on transfection and cytotoxicity was studied. Contrary to the homopolymers, a lower NMVA hydrolysis rate favoured higher transfection efficiency by one order of magnitude but no real influence could be seen on the cytotoxicity. In the following extended study, soft hydrolysis conditions were thus used.

The influence of the molar mass was estimated with three copolymers possessing the same N/M composition close to 50/50 and a similar hydrolysis rate, with molar mass from 10 000 to 52 000 g/mol. High transfection efficiencies were reached with the three copolymers although better efficiency was obtained with molar mass inferior to 50 000g/mol. No real trend could be noticed in cytotoxicity.

Another studied parameter was the copolymer N/M composition. A series of copolymers with increasing VAAm fractions, from zero to one (0%, 25%, 50%, 75% and 100%) were evaluated. Generally, transfection efficiency was similar to PMVAAm unless more than 50% of VAAm were introduced. It was in the same order as 1PEI: 10⁹ RLU/mg proteins and similar to the PMVAAm homopolymer. In contrast, no real influence of the composition on the cytotoxicity was observed. In general, the P(VAAm-co-MVAAm) copolymers were less toxic than 1PEI, with a viability higher than 60%.

The compound 6 was also evaluated on other cells lines, as detailed in example 2. It reached a transfection efficiency around 10⁷ RLU/mg proteins for the muscular and pulmonary cells, in the same range as 1PEI, and 10⁶ RLU/mg on dendritic cells, which was one order better than with 1PEI. On all types of cells (except the formerly tested HeLa), the copolymers reached higher transfection than the PMVAAm homopolymers (increase by one order). Moreover, in all types of cells, the viability was far better than for 1PEI (> 70% versus 20 to 40%, respectively).

The transfection efficiency and the cytotoxicity were also evaluated by FACS with pCMV-EGFP on C2C12, DC2.4 and fibroblast cells. On muscular and dendritic cells, compound 6 had better mean fluorescence intensity (MFI) (150 and 380) than 1PEI (120 and 220) but fewer cells were transfected (4% versus 9% respectively). For the fibroblast, the MFI was around 120 with 20% of cells transfected. Concerning the cytotoxicity, compound 6 was far less toxic than 1PEI. Particularly for C2C12 and DC2.4, excellent viabilities between 55% and 75% were measured versus less than 20% for 1PEI.

A series of polymers according to formula (I) in the form of a statistical copolyvinylamine containing primary and/or secondary amines and vinylimidazole (VIm) moieties was prepared by copolymerization of NVA and/or NMVA with VIm by FRP followed by acidic hydrolysis, as described in example 2. They are referred to as compounds 11, 12 and 13. The conditions of hydrolysis were 2N HCl/120°C/14h, leading to 65% of hydrolysis of NVA and 20 to 30% of NMVA, and a global hydrolysis rate ranging from 65% to 90% in the terpolymer while conditions of 6N HCl, 120°C, 64h gave a hydrolysis level of 84% for NMVA in the copolymer. The characteristics of the copolymers before and after hydrolysis are listed in Table 9. The F_{NVA} and F_{NMVA} values given in Table 9 represent the fractions of the respective monomers in the polymers after hydrolysis.

**TABLE 9: CHARACTERISTICS OF POLYMERS USED IN EXAMPLE 5, BEFORE AND AFTER HYDROLYSIS**

| | | Polymers before hydrolysis | | Polymers after hydrolysis | | | | | |
|---|---|---|---|---|---|---|---|---|---|
| Compound | Reference | Monomers | DP(NVA) - (NMVA)- (VIm) | NVA %hydrol. | NMVA %hydrol. | F_{NVA} | F_{NMVA} | F_{Vim} | Mₙ kg/mol |
| 11 | P(M245-V60) | NMVA - VIm | 245-60 | - | 84 | - | 0.63 | 0.19 | 27.5 |
| 12 | P(N120-M130-V130) | NVA - NMVA - VIm | 120-130-130 | 90 | 97 | 0.03 | 0.01 | 0.34 | 25.6 |
| 13 | P(N170-M90-V55) | NVA - NMVA - VIm | 170-90-55 | 90 | 93 | 0.057 | 0.02 | 0.18 | 18.9 |

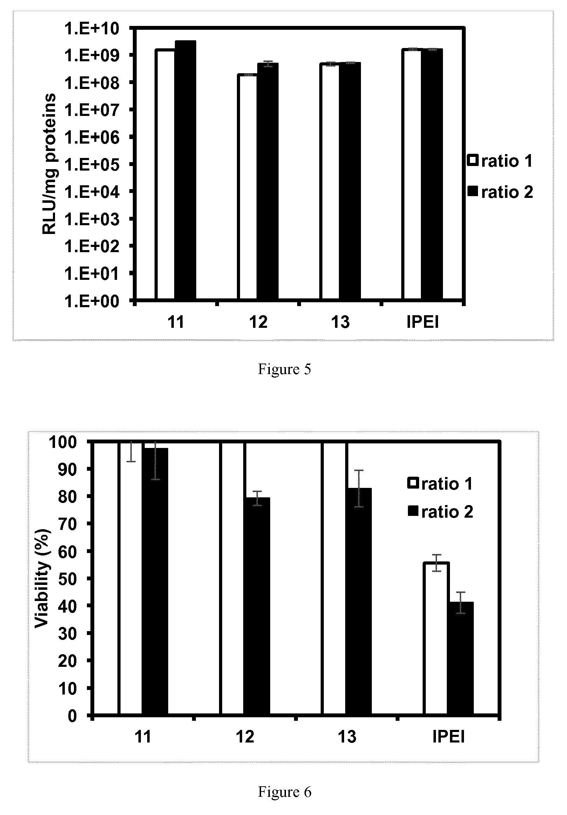

Polyplexes were prepared at different weight ratios, as described in previous examples. Characteristics are shown in Table 10 and results of transfection efficiency and viability in Figs 5 and 6 respectively.

**TABLE 10: CHARACTERISTICS OF POLYPLEXES FORMED IN EXAMPLE 5**

| | Polyplexes | | | | |
|---|---|---|---|---|---|
| Compound | Ratio | pDNA/polymer weight ratio | Phosphate/amine molar ratio | Hydrodynamic diameter (nm) | ζ potential (mV) |
| 11 | 1 | 1/6 | 1/9 | 155 | +48 |
| 11 | 2 | 1/8 | 1/23 | 100 | +48 |
| 12 | 1 | 1/1 | 1/2 | 470 | +42 |
| 12 | 2 | 1/3 | 1/5 | 3950 | +49 |
| 13 | 1 | 1/1 | 1/1 | 125 | +38 |
| 13 | 2 | 1/3 | 1/3 | 140 | +42 |

The transfection performances were tested as described in previous examples. Compound 11, a copolymer P(MVAAm-co-VIm) reached a transfection efficiency of more than 10⁹ RLU/mg. Compounds 12 and 13, terpolymers, also gave very positive results, approaching 10⁹ RLU/mg. All different compounds exhibited cell viability which was always superior to the reference 1PEI (> 80% versus 40-50%).

Compound 11 was also tested on other cells lines, as described previously. The compound reached the same order of transfection efficiency as 1PEI (around 10⁷ RLU/mg proteins) and was better than compound 2 by one order of magnitude. The cytotoxicity was also better than the reference (1PEI) and was identical to that of compound 2.

### COMPARATIVE EXAMPLE 6

To confirm the surprising influence of these residual amide functions, a second series of well-defined polymers PVAm, which did not contain amide functions, was synthesized. A polymer PVAm is a polymer of formula (I) wherein m represents zero such that the polymer only comprises groups of formula (IA). These PVAms were obtained from poly(*N*-vinylphthalimide) (NVPi) made by controlled polymerization (RAFT) followed by the hydrazinolysis of the phthalimide functions.

The synthesis of O-ethyl-S-(1-ethoxycarbonyl) ethyl dithiocarbonate (CTA) was performed according to [6] by mixing ethyl 2-bromopropanoate in ethanol and adding portion-wise potassium ethyl xanthogenate at 0°C. Then the reaction took place at room temperature (∼20°C) overnight. After complete conversion, the product was extracted by Et₂O/ pentane 2/1, washed with H₂O and dried over MgSO₄. The solvents were removed under reduced pressure in order to obtain the desired product as an orange liquid.

According to ref [7], NVPi (5 g, 28.9 mmol) was dissolved in a solution of DMF (14 mL), containing AIBN (23.5 mg, 0.143 mmol) and CTA (132.1 mg, 0.594 mmol). The mixture was placed in a flask equipped with a magnetic stirring bar and the solution was degassed by Argon bubbling for 30 minutes. Then the polymerization was performed at 60°C for 24h. After cooling, the polymer was centrifuged to remove a black precipitate, then was precipitated in Et₂O, filtrated and dried. The resulting PNVPi was analyzed by SEC and 1H NMR, dn/dcPNVPi in DMF = 0.13, *M*nSEC DMF LS = 12.9 kg/mol, *M*nSEC DMF cal PMMA = 4.6 kg/mol, ÐSEC DMF cal PS = 1.6. Similar experiments were carried out varying the initial [NVPi]/[AIBN]/[CTA] molar ratio and the reaction time in order to produced PNVPi of different molar masses (Table 11).

Subsequently, the hydrazinolysis of PNVPi was performed. PNVPi (5 g, 28.9 mmol equal to 28.9 mmol of NVPi) was dissolved in 1,4-dioxane/MeOH 1/2 (100 mL) and monohydrated hydrazine (33 mL, 1078 mmol) was added under inert atmosphere. The reaction was performed at 65°C overnight under stirring. After cooling, the solvents were removed under reduced pressure. In order to obtain the desired polyvinylamine, the resulting solution was treated with acid (HCl 6N). The addition of acid permits the release of 2,3-dihydro-1,4-phthalazinedione phthalhydrazine bond to the amine functions. The final polymer was dialysed against pure water for two days, maintaining the final water at pH 7, and was lyophilized. The resulting PVAm was analyzed was analyzed by 1H NMR in D2O and EA. Hydrolysis yields were determined by EA using the C/N content ratio in the polymer before and after acidic treatment (Table 11). Results of transfection efficiency are presented in Fig. 9.

**TABLE 11: CHARACTERISTICS OF POLYMERS USED IN COMPARATIVE EXAMPLE, BEFORE AND AFTER HYDROLYSIS**

| | Polymers before hydrolysis | | Polymers after hydrolysis | |
|---|---|---|---|---|
| Compound | Monomer composition | DP | % hydrozinolysis | Mₙ kg/mol |
| PN-R50 | N-Vinylphtalimide | 48 | 99 | 3.5 |
| PN-R75 | N-Vinylphtalimide | 74 | n.d. | 4.9 |
| PN-R170 | N-Vinylphtalimide | 167 | 98 | 7.6 |
| PN-R200 | N-Vinylphtalimide | 200 | >99 | 8.7 |

**TABLE 12: CHARACTERISTICS OF POLYPLEXES FORMED IN COMPARATIVE EXAMPLE**

| | Polyplexes | | | |
|---|---|---|---|---|
| Compound | pDNA/polymer weight ratio | Phosphate/amine molar ratio | Hydrodynamic diameter (nm) | ζ potential (mV) |
| PN-R50 | 1/1 | 1/7 | 3490 | +11 |
| PN-R50 | 1/3 | 1/22 | 150 | +37 |
| PN-R75 | 1/2 | 1/8 | 120 | +24 |
| PN-R75 | 1/4 | 1/16 | 100 | +22 |
| PN-R170 | 1/1 | 1/7 | 460 | +40 |
| PN-R170 | 1/3 | 1/21 | 140 | +44 |
| PN-R200 | 1/1 | 1/7 | 90 | +23 |
| PN-R200 | 1/3 | 1/22 | 130 | +40 |

Compared to the PVAm obtained from PNVA, the transfection efficiency for this type of polymer was significantly lower than with PVAAm obtained by acidic hydrolysis (10⁵ vs.10⁸ RLU/mg proteins for a comparable molar mass, compound 1 vs. PN-R170). This result highlights the importance of residual amide functions along the polymer chains. Only 5 to 10 % of residual amides improved the transfection efficiency by a factor of 10².

### References

2. Wolfert, M.A., et al., Polyelectrolyte Vectors for Gene Delivery: Influence of Cationic Polymer on Biophysical Properties of Complexes Formed with DNA. Bioconjugate Chemistry, 1999.10(6): p. 993-1004.
3. Dréan, M., et al., Far beyond primary poly(vinylamine)s through free radical copolymerization and amide hydrolysis. Polymer Chemistry, 2016, 7, 69-78.
4. Debuigne, A., et al., Key Role oƒ Intramolecular Metal Chelation and Hydrogen Bonding in the Cobalt-Mediated Radical Polymerization oƒ N-Vinyl Amides. Chemistry - A European Journal, 2012.18(40): p. 12834-12844.
5. Destarac, M., et al., Macromolecular Design via the Interchange oƒ Xanthates (MADIX): Polymerization oƒ Styrene with O-Ethyl Xanthates as Controlling Agents. Macromolecular Chemistry and Physics, 2002. 203(16): p. 2281-2289.
6. Chikhaoui-Grioune, D., et al., Controlled radical polymerization oƒ N-vinylphthalimide using carboxyl-terminated trithiocarbonate as RAFT agent and preparation of microfibers via electrospinning technique. Journal of Applied Polymer Science, 2010.117(2): p. 1005-1012.
7. Maki, Y., H. Mori, and T. Endo, Controlled RAFT Polymerization oƒ N-Vinylphthalimide and its Hydrazinolysis to Poly(vinyl amine). Macromolecular Chemistry and Physics, 2007. 208(24): p. 2589-2599.

## Claims

1. A polyplex comprising:
(a) at least one polymer of formula (I) comprising at least one set of repeating units of formula: 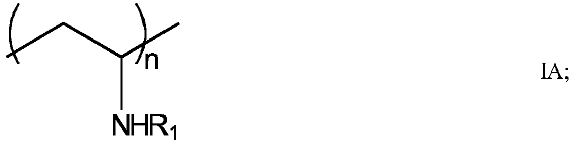 and 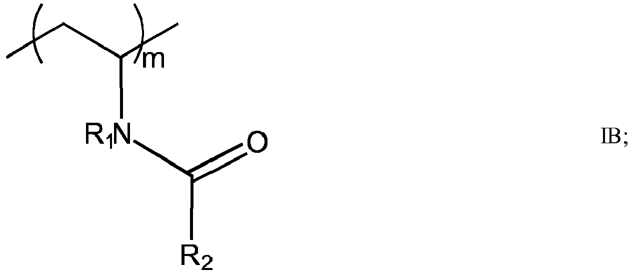
wherein for each set of repeating units R₁ represents either a hydrogen atom or a straight or branched chain alkyl group having from 1 to 20 carbon atoms and R₂ represents a straight or branched chain alkyl group having from 1 to 20 carbon atoms,
wherein n and m each represent an integer of from 1 to 5000,
wherein the sum of n and m is from 10 to 250 when R₁ represents a hydrogen atom and when the polymer of formula (I) contains only one set of repeating units; and
(b) at least one nucleic acid.

2. A polyplex as defined in Claim 1 wherein the polymer of formula (I) comprises one set of repeating units wherein R₁ represents a straight or branched chain alkyl group having from 1 to 20 carbon atoms.

3. A polyplex as defined in Claim 1 or Claim 2 wherein n represents an integer which is greater than the integer represented by m.

4. A polyplex as defined in any one of the preceding Claims wherein the polymer of formula (I) is a copolymer comprising one or more additional repeating units.

5. A polyplex as defined in Claim 4 wherein the one or more additional repeating units comprise a set of repeating units (IA') and (IB') wherein R₁ represents a different substituent to the set of repeating units (IA) and (IB).

6. A polyplex as defined in Claim 4 or Claim 5 wherein the one or more additional units comprise a repeating unit of formula: 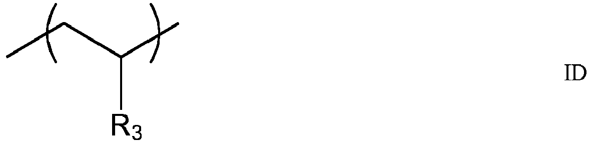 wherein R₃ represents an optionally substituted alkyl, aryl or heterocycle, alcohol, ester or amino-acid containing group; and/or a repeating unit of formula: 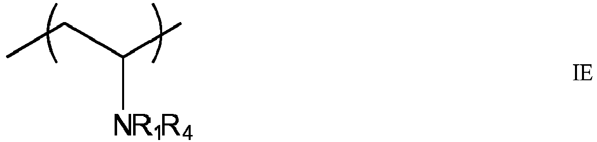 wherein R₁ is as defined in Claim 1 or Claim 2 and R₄ represents a hydrogen atom or an optionally substituted alkyl, aryl or heterocycle group or R₁ and R₄ together form a heterocycle group.

7. A polyplex as defined in any one of the preceding Claims wherein the polymer of formula (I) has a hydrolysis level of from 50% to 97%, preferably from 70 to 95%.

8. A polyplex as defined in any one of the preceding Claims wherein the polymer of formula (I) is in cationic form.

9. A polyplex as defined in any one of the preceding Claims wherein the weight ratio of (a) the at least one nucleic acid to (b) the polymer of formula (I) is from 1:1 to 1:20.

10. A polyplex as defined in any one of the preceding Claims which has a size which is less than 1 µm.

11. A polyplex as defined in any one of the preceding Claims which is positively charged.

12. A polyplex as defined in any one of the preceding claims wherein the at least one nucleic acid comprises a DNA and/or RNA fragment and/or a single or double-stranded, linear or circular, natural or synthetic, modified or unmodified fragment or a portion of a nucleic acid.

13. A method for preparing a polymer of formula (I) for use in a polyplex as defined in any one of the preceding Claims wherein the method comprises one or more of the following steps:
(i) polymerization of a monomer of formula (IC) 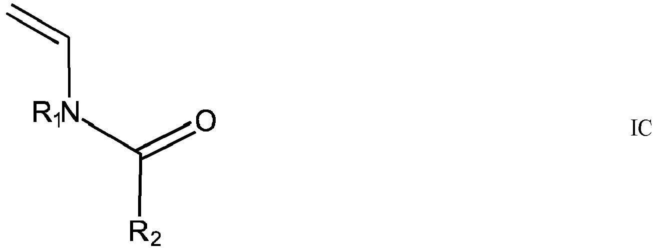 wherein R₁ and R₂ are as defined in Claim 1 followed by hydrolysis, and/or
(ii) polymerization of a monomer of formula (IC) followed by reduction of the amide function; preferably the method comprises step (i) and optionally step (ii); preferably the monomer of formula (IC) is N-vinylacetamide, N-methyl-N-vinylacetamide, N-ethyl-N-vinylacetamide.

14. A polyplex as defined in any one of Claims 1 to 12 wherein the polymer of formula (I) is obtained by the method defined in Claim 13.

15. A method for preparing a polyplex which method comprises the step of combining at least one nucleic acid with at least one polymer of formula (I) as defined in any one of Claims 1 to 8 or Claim 14.

16. A method for transferring at least one nucleic acid into a cell which method comprises the step of contacting the cell with at least one polyplex as defined in any one of Claims 1 to 12 or Claim 14.

17. A pharmaceutical composition comprising a polyplex as defined in any one of Claims 1 to 12 or Claim 14 and an adjuvant.

18. Use of a polyplex as defined in any one of Claims 1 to 12 or Claim 14 or of a pharmaceutical composition as defined in Claim 17 in transferring at least one nucleic acid into a cell.

19. Use of a polymer of formula (I) as defined in any one of claims 1 to 8 in transferring at least one nucleic acid into a cell.
